# EUROPEAN PATENT APPLICATION

(11) **EP 4 386 009 A1**
(43) Date of publication of application: **19.06.2024**
(21) Application number: 22855430.9
(22) Date of filing: 09.08.2022
(51) Int. Cl.: C07K 19/00, A61P 35/00

(54) **BISPECIFIC FUSION POLYPEPTIDE AND APPLICATION THEREOF**

(30) Priority: 09.08.2021 CN 202110910605
(71) Applicant: Yuanpu Biotechnology (Wuhan) Co., Ltd., Wuhan, Hubei 430074 (CN)
(72) Inventor: DONG, Sicong, Chengdu, Sichuan 610000 (CN); ZHANG, Hui, Chengdu, Sichuan 610000 (CN); SUN, Wenjie, Chengdu, Sichuan 610000 (CN); LIU, Yi, Chengdu, Sichuan 610000 (CN); FENG, Yang, Chengdu, Sichuan 610000 (CN)
(74) Representative: Kilger, Ute
(86) International application number: PCT/CN2022/111142
(87) International publication number: WO 2023/016449

(57) **Abstract**

A bispecific fusion polypolypeptide that binds to vascular endothelial growth factor (VEGF) A and VEGFC is provided, comprising a first domain that specifically recognizes VEGFA and a second domain that specifically recognizes VEGFC. Also provided is a method for treating or preventing diseases related to VEGFA or VEGFC by the described bispecific fusion polypeptide.

## Description

### Technical Field

The present invention pertains to the field of fusion polypeptide, and particularly, to bispecific fusion polypeptide for binding both VEGFA and VEGFC, and the application thereof.

### Background Art

Vasculogenesis refers to the formation of new vessels from endothelial precursor cells or vasculoblast cells. Angiogenesis refers to the process in which small blood vessels are formed by the proliferation and migration of endothelial cells from existing blood vessels in tissues. Both vasculogenesis and angiogenesis are crucial in development and subsequent physiological homeostasis, but may be pathogenic in cancer and various ophthalmic diseases.

A vascular endothelial growth factor (VEGF) family, which is very important for both vasculogenesis and angiogenesis, has been identified. The VEGF family comprises VEGFA, VEGFB, VEGFC, VEGFD, VEGFE and placental growth factor (PLGF). VEGF receptor (VEGFR) mainly comprises three subtypes: VEGFR1, VEGFR2 and VEGFR3. Different types of VEGF bind with their respective receptor (VEGFR) subtypes, leading to VEGFR phosphorylation and exerting corresponding biological effects. Therefore, blocking the binding of VEGF to its receptor VEGFR can effectively inhibit proliferation of endothelial cells, vascular proliferation and leakage, thereby achieving the goal of treating diseases associated to endothelial cell proliferation and angiogenesis.

### Summary of the Invention

In order to solve one of the technical problems existing in the art, the present invention provides a novel fusion polypeptide, which can effectively block the binding of various vascular endothelial growth factors (VEGFs) to their receptor VEGFRs, thereby inhibiting proliferation of endothelial cells, vascular proliferation and leakage.

In an aspect, the present invention provides a bispecific fusion polypeptide binding both vascular endothelial growth factor (VEGF) A and VEGFC, wherein the bispecific fusion polypeptide comprises: a first domain specifically recognizing VEGFA, and a second domain specifically recognizing VEGFC.

In some embodiments of the present invention, the first domain can be a vascular endothelial growth factor receptor (VEGFR) specifically recognizing VEGFA or a functional fragment thereof. In some embodiments of the present invention, the first domain can be an antibody specifically recognizing VEGFA or a functional fragment thereof.

In some embodiments of the present invention, the second domain can be VEGFR specifically recognizing VEGFC or a functional fragment thereof. In some embodiments of the present invention, the second domain can be an antibody specifically recognizing VEGFC or a functional fragment thereof.

In some embodiments of the present invention, the first domain may comprise immunoglobulin (Ig)-like domain 2 of VEGFR1 and Ig-like domain 3 of VEGFR2. In some embodiments of the present invention, the first domain may comprise an antibody Fab fragment, a Fab' fragment, a F(ab')₂ fragment, an Fv fragment, a scFv fragment, a nanoantibody, a heavy chain variable region(VH) fragment or a light chain variable region (VL) fragment specifically binding VEGFA. In some embodiments of the present invention, the first domain may comprise Ig-like domain 2 of VEGFR1, Ig-like domain 3 of VEGFR2 and Ig-like domain 4 of VEGFR2.

In some embodiments of the present invention, the second domain comprises Ig-like domain 1, Ig-like domain 2 and Ig-like domain 3 of VEGFR3. In some embodiments of the present invention, the second domain comprises an antibody Fab fragment, a Fab' fragment, a F(ab')₂ fragment, an Fv fragment, a scFv fragment, a nanoantibody fragment, a VH fragment or a VL fragment specifically binding to VEGFA.

In some embodiments of the present invention, the bispecific fusion polypeptide further comprises a third domain, wherein the third domain comprises an Fc region of an immunoglobulin. In some embodiments of the present invention, the immunoglobulin is selected from IgA, IgG, IgM, IgD and IgE. In some embodiments of the present invention, the immunoglobulin is IgG. In some embodiments of the present invention, the immunoglobulin is IgG1, IgG2, IgG3 or IgG4.

In some embodiments of the present invention, the first domain, the second domain and/or third domain connect to each other directly or via a linker.

In some embodiments of the present invention, the bispecific fusion polypeptide comprises a first domain, a second domain and a third domain. In some embodiments of the present invention, the bispecific fusion polypeptide comprises the first domain, the second domain and the third domain connected by any means. In some embodiments of the present invention, the bispecific fusion polypeptide comprises, from the N-terminal to the C-terminal, the first domain-the second domain-the third domain. In some embodiments of the present invention, the bispecific fusion polypeptide comprises, from the N-terminal to the C-terminal, the first domain-the third domain-the second domain. In some embodiments of the present invention, the bispecific fusion polypeptide comprises, from the N-terminal to the C-terminal, the second domain-the first domain-the third domain. In some embodiments of the present invention, the bispecific fusion polypeptide comprises, from the N-terminal to the C-terminal, the second domain-the third domain-the first domain. In some embodiments of the present invention, the bispecific fusion polypeptide comprises, from the N-terminal to the C-terminal, the third domain-the first domain-the second domain. In some embodiments of the present invention, the bispecific fusion polypeptide comprises, from the N-terminal to the C-terminal, the third domain-the second domain-the first domain.

In some embodiments of the present invention, the bispecific fusion polypeptide comprises the first domain and the third domain. In some embodiments of the present invention, the first domain is connected to the N-terminal of the third domain directly or via a linker, or vice versa.

In some embodiments of the present invention, the bispecific fusion polypeptide comprises the first domain and the second domain. In some embodiments of the present invention, the first domain is connected to the N-terminal of the second domain directly or via a linker, or vice versa.

In some embodiments of the present invention, the bispecific fusion polypeptide comprises the second domain and the third domain. In some embodiments of the present invention, the second domain is connected to the N-terminal of the third domain directly or via a linker, or vice versa.

In some embodiments of the present invention, the Fc region of the immunoglobulin has an amino acid sequence as shown in any one of SEQ ID NOs: 12-15, or an amino acid sequence having at least about 85%, 90%, 95%, 98%, 99% or 100% sequence identity to any one of SEQ ID NOs: 12-15.

In some embodiments of the present invention, the linker comprises at least 6 amino acids. In some embodiments of the present invention, the linker has an amino acid sequence as shown in SEQ ID NO: 7 or SEQ ID NO: 8, or an amino acid sequence with one, two or three amino acids insertion, substitution or deletion compared to the amino acid sequence as shown in SEQ ID NO: 7 or 8.

In some embodiments of the present invention, the first domain may comprise an amino acid sequence as shown in SEQ ID NO: 1 or an amino acid sequence having at least about 85%, 90%, 95%, 98%, 99% or 100% sequence identity to SEQ ID NO: 1, and an amino acid sequence as shown in SEQ ID NO: 2 or an amino acid sequence having at least about 85%, 90%, 95%, 98%, 99% or 100% sequence identity to SEQ ID NO: 2. In some embodiments of the present invention, the first domain may comprise an amino acid sequence as shown in SEQ ID NO: 16 or an amino acid sequence having at least about 85%, 90%, 95%, 98%, 99% or 100% sequence identity to SEQ ID NO: 16. In some embodiments of the present invention, the first domain comprises an amino acid sequence as shown in SEQ ID NO: 9 or an amino acid sequence having at least about 85%, 90%, 95%, 98%, 99% or 100% sequence identity to SEQ ID NO: 9.

In some embodiments of the present invention, the second domain comprises: an amino acid sequence as shown in SEQ ID NO: 3, an amino acid sequence corresponding to SEQ ID NO: 3 but with an amino acid at position 75 not being asparagine, or an amino acid sequence having at least about 85%, 90%, 95%, 98%, 99% or 100% sequence identity to SEQ ID NO: 3; an amino acid sequence as shown in SEQ ID NO: 4 or an amino acid sequence having at least about 85%, 90%, 95%, 98%, 99% or 100% sequence identity to SEQ ID NO: 4 ; and an amino acid sequence as shown in SEQ ID NO: 5 or an amino acid sequence having at least about 85%, 90%, 95%, 98%, 99% or 100% sequence identity to SEQ ID NO: 5.

In some embodiments of the present invention, in the amino acid sequence corresponding to SEQ ID NO: 3 but with an amino acid at position 75 not being asparagine, the amino acid at position 75 is replaced with glutamine, aspartic acid, glutamic acid, arginine or lysine. In some embodiments of the present invention, the amino acid sequence corresponding to SEQ ID NO: 3 but with an amino acid at position 75 not being asparagine is an amino acid sequence as shown in SEQ ID NO: 6 .

In some embodiments of the present invention, the second domain comprises: an amino acid sequence as shown in SEQ ID NO: 10, an amino acid sequence corresponding to SEQ ID NO: 10 but with an amino acid at position 80 not being asparagine, or an amino acid sequence having at least about 85%, 90%, 95%, 98%, 99% or 100% sequence identity to SEQ ID NO: 10. In some embodiments of the present invention, in the amino acid sequence corresponding to SEQ ID NO: 10 but with an amino acid at position 80 not being asparagine, the amino acid at position 80 is replaced with glutamine, aspartic acid, glutamic acid, arginine or lysine. In some embodiments of the present invention, the amino acid sequence corresponding to SEQ ID NO: 10 but with an amino acid at position 80 not being asparagine is an amino acid sequence as shown in SEQ ID NO: 11 .

In some embodiments of the present invention, the bispecific fusion polypeptide comprises an amino acid sequence as shown in SEQ ID NO: 17 or an amino acid sequence having at least about 85%, 90%, 95%, 98%, 99% or 100% sequence identity to SEQ ID NO: 17. In some embodiments of the present invention, the bispecific fusion polypeptide comprises an amino acid sequence as shown in SEQ ID NO: 18 or an amino acid sequence having at least about 85%, 90%, 95%, 98%, 99% or 100% sequence identity to SEQ ID NO: 18. In some embodiments of the present invention, the bispecific fusion polypeptide comprises an amino acid sequence as shown in SEQ ID NO: 19 or an amino acid sequence having at least about 85%, 90%, 95%, 98%, 99% or 100% sequence identity to SEQ ID NO: 19. In some embodiments of the present invention, the bispecific fusion polypeptide comprises an amino acid sequence as shown in SEQ ID NO: 20 or an amino acid sequence having at least about 85%, 90%, 95%, 98%, 99% or 100% sequence identity to SEQ ID NO: 20. In some embodiments of the present invention, the bispecific fusion polypeptide comprises an amino acid sequence as shown in SEQ ID NO: 21 or an amino acid sequence having at least about 85%, 90%, 95%, 98%, 99% or 100% sequence identity to SEQ ID NO: 21. In some embodiments of the present invention, the bispecific fusion polypeptide comprises an amino acid sequence as shown in SEQ ID NO: 22 or an amino acid sequence having at least about 85%, 90%, 95%, 98%, 99% or 100% sequence identity to SEQ ID NO: 22. In some embodiments of the present invention, the bispecific fusion polypeptide comprises an amino acid sequence as shown in SEQ ID NO: 23 or an amino acid sequence having at least about 85%, 90%, 95%, 98%, 99% or 100% sequence identity to SEQ ID NO: 23. In some embodiments of the present invention, the bispecific fusion polypeptide comprises an amino acid sequence as shown in SEQ ID NO: 24 or an amino acid sequence having at least about 85%, 90%, 95%, 98%, 99% or 100% sequence identity to SEQ ID NO: 24.

In another aspect, the present invention provides an isolated nucleic acid molecule, which encodes the bispecific fusion polypeptide of the present invention.

In yet another aspect, the present invention provides a nucleic acid delivery vector, which comprises the isolated nucleic acid molecule of the present invention. According to some embodiments of the present invention, the nucleic acid delivery vector may be derived from adenovirus, adeno-associated virus, lentivirus or other acceptable nucleic acid delivery vectors.

In yet another aspect, the present invention provides a bispecific binding molecule for vascular endothelial growth factor (VEGF) A and VEGFC, wherein the bispecific binding molecule comprises a dimer formed by the bispecific fusion polypeptide of the present invention. In some embodiments of the present invention, the fusion polypeptide of the present invention forms a homodimer. In some embodiments of the present invention, the fusion polypeptide of the present invention forms a heterodimer.

In some embodiments of the present invention, the bispecific fusion polypeptide of the present invention comprises a first domain, a second domain and a third domain, wherein the third domain comprises an Fc region of an immunoglobulin. In some embodiments of the present invention, the bispecific fusion polypeptide comprising, from the N-terminal to the C-terminal, the first domain-the second domain-the third domain, forms a dimer. In some embodiments of the present invention, the bispecific fusion polypeptide comprising, from the N-terminal to the C-terminal, the first domain-the third domain-the second domain, forms a dimer. In some embodiments of the present invention, the bispecific fusion polypeptide comprising, from the N-terminal to the C-terminal, the second domain-the first domain-the third domain, forms a dimer. In some embodiments of the present invention, the bispecific fusion polypeptide comprising, from the N-terminal to the C-terminal, the second domain-the third domain-the first domain bispecific fusion polypeptide, forms a dimer. In some embodiments of the present invention, the bispecific fusion polypeptide comprising, from the N-terminal to the C-terminal, the third domain-the first domain-the second domain, forms a dimer. In some embodiments of the present invention, the bispecific fusion polypeptide comprising, from the N-terminal to the C-terminal, the third domain-the second domain-the first domain, forms a dimer.

In yet another aspect, the present invention provides a host cell, which comprises the isolated nucleic acid molecule of the present invention.

In yet another aspect, the present invention provides a pharmaceutical composition. In some embodiments of the present invention, the pharmaceutical composition comprises the bispecific fusion polypeptide of the present invention, as well as a pharmaceutically acceptable carrier. In some embodiments of the present invention, the pharmaceutical composition comprises the isolated nucleic acid molecule of the present invention, as well as a pharmaceutically acceptable carrier. In some embodiments of the present invention, the pharmaceutical composition comprises the nucleic acid delivery vector of the present invention, as well as a pharmaceutically acceptable carrier. In some embodiments of the present invention, the pharmaceutical composition comprises the bispecific binding molecule of the present invention, as well as a pharmaceutically acceptable carrier.

In some embodiments of the present invention, the pharmaceutical composition is in the form of tablet, fine powder, granule, pill, injection, suspension, powder, emulsion, aerosol, gel, eye drop, sustained release formulation or sustained release implant. In some embodiments, the pharmaceutical composition may be formulated into an injectable formulation. In some embodiments, the formulation is suitable for intravitreal, subcutaneous, intradermal, intramuscular, intravenous, intrathecal, or intraspinal administration.

In yet another aspect, the present invention provides a kit, comprising the pharmaceutical composition of the present invention, wherein the pharmaceutical composition is enclosed in a container. In some embodiments of the present invention, the container is a glass ampoule, a glass vial, a plastic ampoule, a plastic vial, a plastic pouch or a pre-loaded syringe. In some embodiments, the present invention relates to a drug unit dosage form suitable for parenteral administration to human beings, comprising the pharmaceutical composition as described herein in a suitable container. In some embodiments, the suitable container is a pre-loaded syringe. In some embodiments, the pre-loaded syringe comprises an injection needle.

In yet another aspect, the present invention provides a method for treating or preventing diseases associated to VEGFA or VEGFC. The method comprises administering to a subject a therapeutically effective amount of the bispecific fusion polypeptide of the present invention, the isolated nucleic acid molecule of the present invention, the nucleic acid delivery vector comprising the isolated nucleic acid molecule of the present invention, the bispecific binding molecule of the present invention, or the pharmaceutical composition of the present invention.

In some embodiments of the present invention, the disease is selected from angiogenic ophthalmopathy and cancer related indications. In some embodiments of the present invention, the disease is selected from age related macular degeneration, diabetes macular edema, diabetes retinopathy, angiogenic glaucoma, retinal vein occlusion, corneal neovascularization, neovascularization after corneal transplantation, breast cancer, kidney cancer, ovarian cancer, fallopian tube cancer, peritoneal cancer, gastric cancer, colorectal cancer, non-small cell lung cancer, bladder cancer, pancreatic cancer, liver cancer, cervical cancer and glioblastoma.

### Detailed description of the invention

### Definitions

Unless otherwise defined, all scientific and technical terms used herein have the same meanings as those commonly used in the field to which the present invention pertains. For the purpose of interpreting the present description, the following definitions will apply, and where appropriate, terms used in singular form will also include plural form, and vice versa.

Unless otherwise explicitly stated in the context, the expressions "one", "a", and "an" used in this article include plural references. For example, mentioning "a cell" includes multiple such cells and equivalents that are known to those skilled in the art.

As used herein, the term "about" represents a range of ± 20% of the numerical value after the term. In some embodiments, the term "about" represents a range of ± 10% of the numerical value after the term. In some embodiments, the term "about" represents a range of ± 5% of the numerical value after the term.

As used herein, the term "vascular endothelial growth factor" or "VEGF" refers to the vascular endothelial growth factor having 165 amino acids, and the relevant vascular endothelial growth factors having 121, 189 and 206 amino acids (such as those described in, Leung et al., Science 246:1306(1989); and Houck et al., Mol. Endocrin.5:1806(1991)), as well as the naturally occurring allele forms and processed forms of these growth factors. VEGF family comprises VEGFA, VEGFB, VEGFC, VEGFD, VEGFE and placental growth factor (PLGF). VEGF is a factor that promotes the growth of vascular endothelial cells with highly specificity and contributes to increased vascular permeability, extracellular matrix degeneration, vascular endothelial cell migration, proliferation and vascular formation.

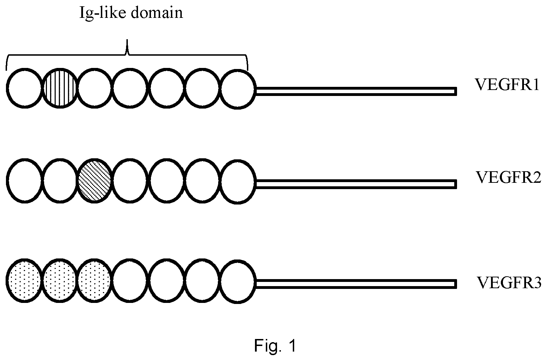

As used herein, the term "vascular endothelial growth factor receptor", "VEGF receptor" or "VEGFR" refers to the cellular receptor of VEGF, which are usually the cell surface receptor found on vascular endothelial cells and a variant thereof retaining the ability of binding VEGF. VEGFR family mainly comprises VEGFR1, VEGFR2 and VEGFR3. Each of these three members of the VEGFR family contains an extracellular domain, a transmembrane domain, and an intracellular tyrosine kinase domain, all having seven immunoglobulin (Ig)-like domains in the extracellular domain (as shown in Fig. 1). The seven immunoglobulin domains are named, from the N-terminus of the protein, as Ig-like domain 1, Ig-like domain 2, Ig-like domain 3, Ig-like domain 4, Ig-like domain 5, Ig-like domain 6, and Ig-like domain 7, respectively. VEGFR binds to VEGF through its extracellular domain, and its Ig-like domain has a high affinity for VEGF. VEGFR1 and VEGFR2 are expressed on most vascular endothelial cells, while VEGFR3 is mainly expressed on lymphatic endothelial cells. By binding to VEGFR1 and VEGFR2, VEGFA can activate intracellular signaling pathways, participate in physiological and pathological processes such as cell proliferation and angiogenesis, not only promoting neovascularization and increased vascular permeability, but also preventing cell apoptosis to maintain blood vessels. It has been reported that the second Ig-like domain (i.e., Ig-like domain 2) of VEGFR1 determines the binding of the receptor with VEGF (Terri Davis Myth et al., The EMBO Journal, vol.15 no.18, pp4919-4927, 1996). VEGFC can bind to VEGFR2 and VEGFR3, mainly used for the regulation of lymphangiogenesis. VEGFB may play a role in non-angiogenic tumors, while VEGFC and VEGFD can play a role in the formation of new blood vessels and new lymphatic vessels in cancer tissues. VEGFE is also a potential angiogenic factor. It has been reported that VEGFR3 can also bind to VEGFD. By specifically binding to the receptor VEGFR1/Flt-1, PLGF activates the signaling pathway, mediate the actions of endothelial cells and stromal cells, and influences the differentiation and maturation of endothelial cells.

As used herein, the term "fusion polypeptide" means that, by genetic expression of polynucleotides encoding proteins or protein synthesis methods, two or more proteins or their fragments are collinearly connected via their respective polypeptide skeletons. Preferably, the polypeptide or fragments thereof are from different sources. In some embodiments, the fusion polypeptide includes polypeptide fragments from different sources, such as from VEGFR1, VEGFR2, VEGFR3, immunoglobulin, or antibody fragments.

As used herein, the term "affinity" or "binding affinity" refers to the strength of the sum of the noncovalent interaction between the individual binding sites of a molecule (such as, the bispecific fusion polypeptide or bispecific binding molecule) and its binding ligand (such as, an antigen). Binding affinity can usually be represented by dissociation constant (K_{D}), which is the ratio of dissociation rate (k_{d}) to binding rate (kₐ), i.e. K_{D}=k_{d}/kₐ. Affinity can be measured using conventional methods known in this field, such as surface plasmon resonance (SPR).

As used herein, the term "specific recognition" or "specific binding" refers to the recognition and binding selectivity of fusion polypeptide or specific binding molecule to the ligand, which can be used to distinguish from unwanted or non-specific binding. In an embodiment, the bispecific fusion polypeptide or bispecific binding molecule of the present invention has a binding strength with unrelated proteins less than about 10% of the binding strength with VEGFA and VEGFC, as measured by, for example, SPR. In some embodiments, the bispecific fusion polypeptide or bispecific binding molecule of the present invention bind to VEGFA and VEGFC with a K_{D} of 10⁻⁷ M or lower, such as 10⁻¹⁰ M to 10⁻¹¹ M.

As used herein, the term "substitution" for amino acid refers to the replacement of at least one amino acid residue in an amino acid sequence by another different "substituting" amino acid residue. As used herein, the term "insertion" for amino acids refers to the incorporation of at least one additional amino acid into an amino acid sequence. Although an insertion typically consists of inserting 1 or 2 amino acid residues, a larger "polypeptide insertion" can also be incorporated, such as inserting about 3-5 or even up to about 10, 15 or 20 amino acid residues. As disclosed above, the inserted residue can be derived from either naturally or non-naturally present amino acid. As used herein, the term "deletion" for amino acid refers to the removal of at least one amino acid residue from an amino acid sequence.

The fusion polypeptide or its fragment disclosed herein may contain a conservative amino acid substitution at one or more amino acid residues, such as at essential or non-essential amino acid residues. "Conservative amino acid substitution" refers to the replacement of an amino acid residue by another amino acid residue having a similar side chain. Definitions for families of amino acid residues having similar side chains have been defined in the art, including those with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g. , alanine, valine, leucine, isoleucine, proline, phenylalanine, methionine, tryptophan), beta-branched side chains (e.g., threonine, valine, isoleucine) and aromatic side chains (e.g. , tyrosine, phenylalanine, tryptophan, histidine). Therefore, in the present description, an essential or non-essential amino acid residue in the fusion polypeptide or linker is preferably replaced with another amino acid residue from the family having the same side chains. In some embodiments, an amino acid chain segment may be replaced with a structurally similar chain segment having different order and/or composition of the family members with similar side chains. Alternatively, in some embodiments, mutations may be randomly introduced along all or part of the encoding sequence, such as through saturation mutagenesis, and the resulting mutants may be incorporated into the fusion polypeptide of the present invention, and then screened for the ability of these polypeptides to bind to the desired target.

As used herein, the term "antibody" includes both complete antibody and any antigen-binding fragment (i.e., "antigen-binding portion", "antigen-binding polypeptide" or "immune binding agent"), or their single chains. "Antibody" is a kind of glycoprotein that contains at least two heavy chains (H) and two light chains (L) connected to each other through disulfide bonds, or its antigen-binding portion. Each heavy chain includes a heavy chain variable (VH) region and a heavy chain constant (CH) region. Each light chain includes a light chain variable (VL) region and a light chain constant (CL) region. The VH and VL regions each contain three regions with highly variable amino acid composition and arrangement sequences, known as the hypervariable region or complementarity determining region (CDR), i.e., CDR1, CDR2, and CDR3. The other regions in the VH and VL regions, except for CDR regions, have relatively conservative amino acid composition and arrangement order, known as the framework region (FR). VH or VL each has four framework regions, represented by FR1, FR2, FR3, and FR4, respectively.

There are five main categories of immunoglobulins, i.e., IgA, IgD, IgE, IgG, and IgM, and these main categories can be further divided into subclasses (isotypes), such as IgG1, IgG2, IgG3, IgG4, IgA1, and IgA2. The heavy chain constant domains corresponding to different categories of immunoglobulins are called α, δ, ε, γ and µ. Different isotypes (κ or λ) of immunoglobulins have substantially same CL length but different CH length. For example, IgG, IgA, and IgD comprise CH1, CH2, and CH3, while IgM and IgE comprise CH1, CH2, CH3, and CH4. A hinge region is located between CH1 and CH2, rich in proline and prone to stretching and bending, thereby changing the distance between antigen binding sites and facilitating antibody binding to antigen epitopes located at different positions. The hinge region may be easily hydrolyzed by papain, pepsin, and other enzymes, producing different hydrolysis fragments. The hydrolysis site of Ig by papain is at the near N-terminals of the two heavy chains connected by disulfide bonds in the hinge region, leading to the cleavage of Ig into two identical Fab segments and one Fc segment. The Fab segment is a fragment antigen binding (Fab) segment composed of one complete VH and CHI domains of both light and heavy chains.

As used herein, the term Fc region of an "antibody", "immunoglobulin" or "Ig" refers to the constant domain or constant region (CH) at the C-terminal of the immunoglobulin heavy chain or its fragments, which is a crystallizable fragment (fragment crystallizable, Fc) composed of the CH2 and CH3 domains of Ig. As used herein, the term "Fc region" includes both wild-type Fc regions and variant Fc regions. The wild-type Fc region represents the same amino acid sequence as that of the naturally occurring Fc region. SEQ ID NO: 9 shows the Fc region of wild-type human IgG1. The term "variant (human) Fc region" refers to an amino acid sequence that differs from the wild-type (human) Fc region by at least one amino acid mutation. In some embodiments, the variant Fc region has at least one amino acid mutation, such as 1 to 10 amino acid mutations, compared to the wild-type Fc region. In some embodiments, the variant Fc region has 1 to 3 amino acid mutations compared to the wild-type Fc region.

As used herein, the term "homodimer" refers to a dimer formed from the fusion polypeptide of the present invention, consisting of two same fusion polypeptides of the present invention. As used herein, the term "heterodimer" refers to a dimer formed from the fusion polypeptide of the present invention, consisting of two different fusion polypeptides of the present invention.

As used herein, the term "individual" or "subject" refers to mammals, including but not limited to human and non-human mammals. For example, mammals include but not limited to domesticated animals (such as cows, horses, dogs, sheep, goats, cats, and dogs), primates (such as humans and monkeys), and rodents (such as rabbits, mice, and rats).

The numerical range used herein should be understood as having already listed all the numbers within that range. For example, the range of from 1 to 20 should be understood as including any number, combination of number, or subrange from the following group: 1, 2, 3, 4, 5, 6, 7, 8, 9, 10, 11, 12, 13, 14, 15, 16, 17, 18, 19 or 20.

As used herein, the term "linker" refers to a natural and/or synthetic (polypeptide) linker composed of linear amino acids. The various domains in the bispecific fusion polypeptide of the present invention can be connected via a linker, where each linker is fused and/or otherwise connected (e.g. via a polypeptide bond) with at least two polypeptides or domains. In some embodiments, the amino acid sequences of the linkers present in the bispecific fusion polypeptide of the present invention are all the same. In other embodiments, the amino acid sequences of at least two linkers present in the bispecific fusion polypeptide of the present invention are different. A linker should have a length suitable for connecting two or more individual domains in such a manner that the different domains it connects can fold correctly and present appropriately, thereby executing their biological activity functions. In different embodiments, the linker has a flexible conformation. Suitable flexible linkers include, for example, those having glycine, glutamine, and/or serine residues. In some embodiments, the amino acid residues in the linker may be arranged as small repeating units of up to 5 amino acids, such as amino acid sequences shown by GGGSGG (SEQ ID NO: 6) or GGGSGGG (SEQ ID NO: 7).

The "percentage (%) sequence identity" relative to a reference amino acid sequence, refers to the percentage of amino acid residues in the candidate sequence that are the same as those in the reference amino acid sequence after sequence alignment and (as needed) introduction of gaps to achieve a maximum percentage sequence identity, without considering any conservative substitutions as part of sequence identity. To determine the sequence identity percentage, sequence alignment can be performed by any of the various means in the art, such as using BLAST, ALIGN, or Megalign (DNASTAR) softwares. Those skilled in the art can determine appropriate parameters for sequence alignment, including any algorithm required to achieve maximum alignment across the entire length of the aligned sequence.

As used herein, the term "pharmaceutically acceptable carrier" refers to the components other than the active ingredient in a drug formulation, which are not toxic to the subject. Pharmaceutically acceptable carriers include, but are not limited to, buffering agents, excipients, stabilizers, or preservatives.

As used herein, the term "treatment" refers to alleviating and/or improving a disorder and/or its associated disease or symptoms, as well as preventing the symptoms of the disorder from deterioration. The expected therapeutic effects include, but are not limited to, preventing the occurrence or recurrence of a disease, alleviating its symptoms, reducing any direct or indirect pathological outcomes of the disease, preventing metastasis, slowing down disease progression, improving or alleviating symptoms, and alleviating or improving prognosis. However, it should be understood that treating a disease or symptom does not require complete elimination of the disease or its associated symptoms.

As used herein, the term "effective amount" refers to the amounts within a necessary dosage and time period to effectively achieve the desired therapeutic or preventive effect.

The following examples and drawings are provided to assist in understanding the present invention. However, it should be understood that these examples and drawings are intended to be illustrative rather than limitative. The actual protection scope of the present invention is defined in the claims. It should further be understood that any modifications or changes may be made to the present invention without departing from the spirit of the present invention.

### Brief Description of the Drawings

Fig. 1 shows the structural diagrams of the three subtypes, i.e., VEGFR1, VEGFR2, and VEGFR3 of the VEGFR family.
Fig. 2 shows the structural diagram of the constructed fused polypeptide chain, wherein the domains that are same as those in Fig. 1 are illustrates as the same pattern, and "*" indicates a mutation in Ig-like domain 1 located in VEGFR3.
Fig. 3 shows a schematic diagram of the expressed fusion polypeptide dimer.
Fig. 4 shows the binding of the bispecific protein of the present invention to VEGFA and VEGFC. (A) the ELISA results of binding of the bispecific proteins BiFpC1, BiFpC2, BiFpC3, BiFpC4, and BiFpC5 of the present invention to VEGFA; (B) the ELISA results of binding of the bispecific protein of the present invention to VEGFC; (C) the ELISA results of binding of the bispecific proteins BiFpC2 and BiFpC6 of the present invention to VEGFA(VA) or VEGFC(VC).
Fig. 5 shows that a simultaneous binding of the bispecific proteins BiFpC2 and BiFpC6 of the present invention to VEGFA and VEGFC. (A) the simultaneous binding of BiFpC2 to VEGFA and VEGFC. (B) the simultaneous binding of BiFpC6 to VEGFA and VEGFC.
Fig. 6 shows inhibition of the bispecific protein of the present invention on HUVEC cell proliferation induced by VEGFA or VEGFC. (A) the inhibition of BiFpC2 on HUVEC cell proliferation induced by VEGFA. (B) the inhibition of BiFpC6 on HUVEC cell proliferation induced by VEGFA. (C) the inhibiton of BiFpC2 on HUVEC cell proliferation induced by VEGFC. (D) the inhibiton of BiFpC6 on HUVEC cell proliferation induced by VEGFC.
Fig. 7 shows the competitive binding of the bispecific protein of the present invention to VEGFA and VEGFC. (A) the competitive binding of BiFpC2 and Eylea to VEGFA. (B) the competitive binding of BiFpC6 and VEGFR2 to VEGFA. (C) the competitive binding of BiFpC2 and VEGFR3-Fc to VEGFC. (D) the competitive binding of BiFpC6 and VEGFR3 to VEGFC.

### Detailed Description of the Embodiments

The present invention is now further described in details in combination with Examples for providing clear and detailed explanation of the purpose, technical solution, and advantages of the present invention. The specific embodiments described herein are only for illustrating, but not for limiting, the present invention. In addition, in the following description, details of well-known structures and techniques have been omitted to avoid unnecessary confusion with the concepts disclosed herein. Such structures and techniques have also been described in many publications, such as Sambrook, J., Fritsch, E.F. and Maniais, T. (1989) Molecular Cloning: A Laboratory Manual, 2nd edition, Cold spring Harbor Laboratory Press.

### Example 1: Construction of expression plasmid

In this example, Ig-like domain 2 of VEGFR1, Ig-like domain 3 of VEGFR2, Ig-like domains 1-3 of VEGFR3, as well as a single chain antibody fragment (scFv) as shown in SEQ ID NO: 16 for VEGFA and human IgG1-Fc were selected to combine as shown in Table 1.

**Table 1: sequence composition of the fusion polypeptides**

| fusion polypeptide | Structures | | | | |
|---|---|---|---|---|---|
| BiFpC1 | R1D2 (SEQ ID NO: 1) | R2D3 (SEQ ID NO: 2) | Fc (SEQ ID NO: 12) | Linker 1 (SEQ ID NO: 7) | R3D1(N517Q)D2D3 (SEQ ID NO: 11) |
| BiFpC2 | R1D2 (SEQ ID NO: 1) | R2D3 (SEQ ID NO: 2) | Linker 1 (SEQ ID NO: 7) | R3D1(N291Q) D2D3 (SEQ ID NO: 11) | Fc (FL) (SEQ ID NO: 13) |
| BiFpC3 chain 1 | R1D2 (SEQ ID NO: 1) | R2D3 (SEQ ID NO: 2) | Fc(T351S/L353A/Y392V) (SEQ ID NO: 14) | | |
| BiFpC3 chain 2 | R3D1(N80Q)D2D3 (SEQ ID NO: 11) | | Fc(T451W) (SEQ ID NO: 15) | | |
| BiFpC4 | R3D1(N80Q)D2D3 (SEQ ID NO: 11) | | Fc (FL) (SEQ ID NO: 13) | Linker 2 (SEQ ID NO: 8) | anti-VEGFA scFv (SEQ ID NO: 16) |
| BiFpC5 chain 1 | anti-VEGFA scFv (SEQ ID NO: 16) | | Fc(T397S/L399A/Y438V) (SEQ ID NO: 14) | | |
| BiFpC5 chain 2 | R3D1(N80Q)D2D3 (SEQ ID NO: 11) | | Fc (T451W) (SEQ ID NO: 15) | | |
| BiFpC6 | R1D2 (SEQ ID NO: 1) | R2D3 (SEQ ID NO: 2) | Linker 1 (SEQ ID NO: 7) | R3D1D2D3 (SEQ ID NO: 10) | Fc (FL) (SEQ ID NO: 13) |

| | | | | | |
|---|---|---|---|---|---|
| Notes: "R1D2" denotes Ig-like domain 2 of VEGFR1. "R2D3" denotes Ig-like domain 3 of VEGFR2. "R3D1D2D3" denotes Ig-like domain 1, Ig-like domain 2 and Ig-like domain 3 of VEGFR3. "Fc" denotes Fc region of human immunoglobulin IgG1. "Fc(FL)" denotes a full-length Fc and is Fc region of human IgG1, having 5 more amino acids "EPKSC" relative to "Fc" and one more disulfide bond in hinge region relative to "Fc". The indicated mutation sites are based on the numbering of amino acids starting from the N-terminus of the fused polypeptide chain on which the mutation is located. | | | | | |

According to the structure shown in the table above, eight fused polypeptide chains were obtained, having the following amino acid sequences:
BiFpC1: SEQ ID NO: 17;
BiFpC2: SEQ ID NO: 18;
BiFpC3 chain 1: SEQ ID NO: 19;
BiFpC3 chain 2: SEQ ID NO: 20;
BiFpC4: SEQ ID NO: 21;
BiFpC5 chain 1: SEQ ID NO: 22;
BiFpC5 chain 2: SEQ ID NO: 23;
BiFpC6: SEQ ID NO: 24.

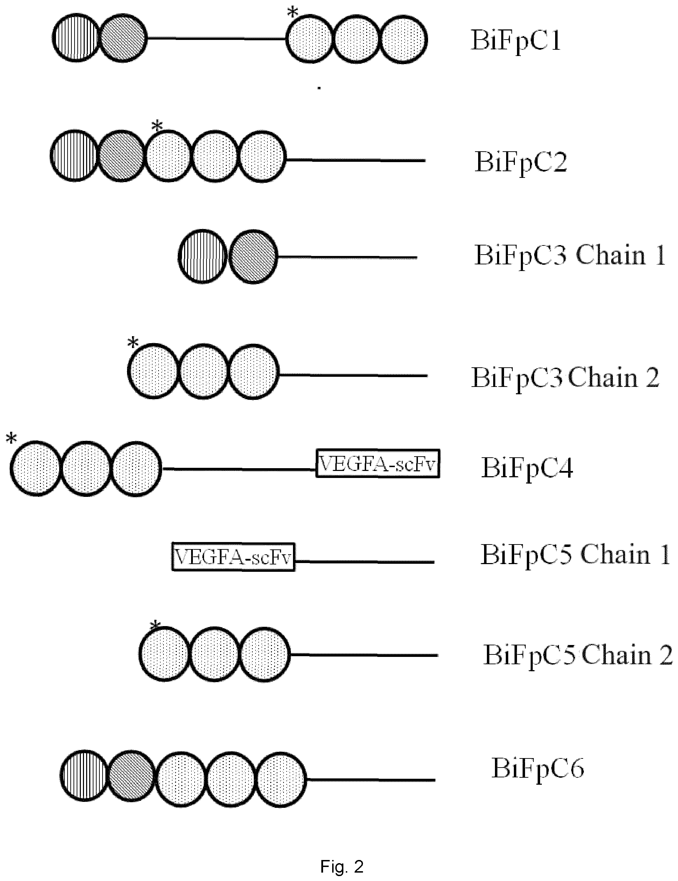

Fig. 2 shows the structural diagrams of these fusion polypeptide chains, "*" in the figure indicates that there is mutations in the amino acids sequence.

Using standard molecular biology techniques (see, for example, Sambrook, J., Fritsch, E.F. and Maniais, T. (1989) Molecular Cloning: A Laboratory Manual, 2nd edition, Cold spring Harbor Laboratory Press), the nucleic acid sequences encoding the above eight fusion polypeptide chains were inserted into a transient expression vector ptt5 or a stable expression vector FTP002, respectively. Specifically, BiFpC1, BiFpC2, BiFpC4 and BiFpC6 were inserted into the multiple cloning sites of EF-1α promoter downstream of vector FTP002; BiFpC3 chain 1, BiFpC3 chain 2, BiFpC5 chain 1 and BiFpC5 chain 2 were inserted into the multiple cloning sites of vector ptt5. Eight plasmid constructs, i.e., FTP002-BiFpC1, FTP002-BiFpC2, ptt5-BiFpC3 chain 1, ptt5-BiFpC3 chain 2, FTP002-BiFpC4, ptt5-BiFpC5 chain 1, ptt5-BiFpC5 chain 2, FTP002-BiFpC6 were obtained. The correctness of the insertion sequence was verified through sequencing

### Example 2: Expression of bispecific proteins

The stable expression vectors FTP002-BiFpC1, FTP002-BiFpC2, FTP002-BiFpC4, and FTP002-BiFpC6 constructed in Example 1 were transfected into the Chinese hamster cell line CHO HD-BIOP3 (Horizon Discovery Ltd., USA) using a cationic polymer transfection reagent (ATG feet solution, Cat. No. ATO00017, AtaGenix Laboratories, Wuhan, China) according to the manufacturer's instructions. Then, the cells were cultured in FectoCHO^{™} CD culture medium (Cat. No.: 716-06L, Polyplus) containing 4mM L-glutamine (Cat. No.: A3704, Applichem). The expression vectors encode the gene of EF-1α promoter and glutamine synthetase (GS). The expression of GS allows the biochemical synthesis of glutamine, which is an amino acid needed for CHO HD-BIOP3 cell growth. After transfection, the cells were subjected to mass selection with 25 µM L-methionine-sulfoximine (MSX, Cat. No.: M111096, Aladdin scientific, China). Stringency of selection may be improved by inhibiting GS with MSX. Cells with expression vector cDNA, which integrated into the transcriptional active region of the host cell genome, were selected against CHO HD-BIOP3 wild-type cells, wherein CHO HD-BIOP3 wild-type cells did not express endogenous levels of GS. The transfected cells were seeded at low density (about 0.8 cells/well) in a 96-well plate to allow for approaching exponential growth of stable expressed cells. The masterwells were screened for bispecific protein expression and subsequently cultured expansively in suspension in serum-free Dynamis medium (Cat. No.: A26175-02, Gibco, USA) to express the bispecific protein.

Transient expression vectors ptt5-BiFpC3 chain 1 and ptt5-BiFpC3 chain 2 were co-transfected into the HEK-293 cell line using a cationic polymer transfection reagent (ATG feet solution, Cat. No. ATO00017, AtaGenix Laboratories, Wuhan, China) according to the manufacturer's instructions. The HEK-293 cells for transient expression of bispecific proteins were cultured in an incubator at 37°C, 95% relative humidity, and 5% CO₂ and harvested when the cell viability decreased to a certain extent (without monoclonal screening and culture). Transient expression vectors ptt5-BiFpC5 chain 1 and ptt5-BiFpC5 chain 2 were treated similarly to express bispecific proteins.

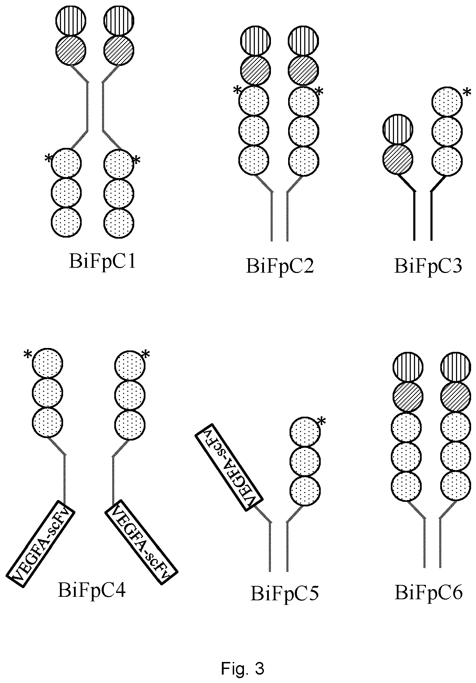

Fig. 3 shows a schematic diagram of the expressed bispecific protein.

### Example 3: Purification of bispecific proteins through affinity chromatography and determination of their expression levels and aggregation characteristics

The bispecific protein expressed in Example 2 was secreted into the culture medium. The culture medium containing the bispecific protein was added to the protein A affinity column Unimab 50 (Cat. No. 17010-050100, Suzhou NanoMicro Technology Co Ltd, China) that has been equilibrated with a phosphate buffer solution (pH 7.4). Then, the bound bispecific protein was eluted with 0.1 M sodium citrate buffer solution (pH 3.4). The solution of the eluted bispecific protein was adjusted with acid (such as 0.1 M citric acid) or base (0.1 M Tris) to a pH that allows the bispecific protein to maintain in a stable state, and then stored at 4°C for detection, or stored at -80°C after packaging.

UV quantification of 1 mL bispecific protein was carried out using UV spectrophotometry according to the Chinese Pharmacopoeia (2020 edition). UV absorbance at 280 nm was recorded and divided by the absorption coefficient of bispecific protein to calculate the concentration of bispecific protein. The expression level of bispecific proteins can be calculated using the formula: bispecific protein expression level (mg/L) = concentration (mg/mL) × elution volume from affinity chromatography (mL) / CHO cell supernatant volume (L). The expression level can also be expressed by determining Titer of cell supernatant, and the experimental method is available from Sound (Chengdu) Biopharmaceuticals Co., Ltd.

In addition, the aggregation characteristics of the expressed bispecific proteins were tested. The expressed candidate bispecific proteins were tested by molecular sieve (SEC) HPLC. The sample to be tested was diluted to a concentration of 1.0 mg/mL and injected 10 µL (10 µg) onto a molecular sieve column (Sepax Zenix-C SEC-300) for assay. The test was performed at the following conditions: flow rate of 0.65 mL/min; column temperature of 35°C, detection wavelength of 214 nm; mobile phase of 200 mM phosphate buffer solution and 300 mM NaCl, pH 7.0. The aggregates, main peaks, and fragments of bispecific protein were tested from the integration of appropriate peaks.

**Table 2: Expression levels and aggregation characteristics of candidate bispecific protein purified by affinity chromatography.**

| bispecific protein | expression level (mg/L) | SEC-HPLC | | |
|---|---|---|---|---|
| | | aggregate % | main peak % | fragment % |
| BiFpC1 | 4.00-6.00 | <10 | >90 | <10 |
| BiFpC2 | 25.00-30.00 | <10 | >90 | <10 |
| BiFpC3 | 4.00-6.00 | >20 | <80 | <10 |
| BiFpC4 | 5.00-7.00 | <10 | >90 | <10 |
| BiFpC5 | 25.00-30.00 | <10 | <60 | >40 |
| BiFpC6 | 30.00-35.00 | <20 | >80 | <10 |

According to the data in Table 2, it can be seen that there are significant differences in expression levels of the bispecific proteins having different structures, wherein BiFpC2, BiFpC5 and BiFpC6 are expressed at a higher level, all above 25 mg/L, while the other bispecific proteins are expressed at a lower level, reaching only about 1/5 of the expression levels of the three former bispecific protein. In addition, samples of bispecific proteins having different structures obtained from affinity chromatography differ significantly from each other in purity. For example, BiFpC5, despite of its high expression level, apparently exhibits a very low main peak purity (the target product can be reflected by the main peak purity in the SEC-HPLC detection results), and the main impurities are fragmented molecules. For another example, BiFpC3 is expressed at a very low level, and contains aggregate impurities in significant amounts (over 20%). The above results all indicate that different designs of molecular structure would lead to dramatically different expression levels and purities.

### Example 4: Test for the binding of bispecific proteins of the present invention to VEGFA and VEGFC

ELISA was carried out in this example to test the binding of the bispecific protein of the present invention to VEGFA and VEGFC.

### 4.1 Test for the binding of bispecific proteins to VEGFA

A 96-well plate was coated with 0.2 µg/mL human VEGFA (Cat. No.: Z03073-10µg, Genscript, China) in a phosphate buffer solution (1× PBS) at 50 µL/well, and then sealed at 4°C overnight for coating. Each well was washed twice with a washing buffer solution 1× PBST (8.01g NaCl, 0.2g KCl, 1.42g Na₂HPO₄, 0.27g KH₂PO₄, 0.05% Tween-20, pH7.4), added with PBST containing 1% bovine serum albumin (BSA, BIOFROXX) at 200 µL/well for blocking. After the 96-well plate was sealed and incubated at 37°C for 2 hours, each well was washed twice with 1× PBST to remove a blocking agent. And then added with bispecific protein of the present invention, i.e., BiFpC1, BiFpC2, BiFpC3, BiFpC4 and BiFpC5, as well as positive control Eylea, as 4-fold series dilution (diluted in 1× PBS), at 50 µL/well. After incubation at 37°C for 1 hour, each well was washed three times with 1 × PBST to remove any unbound bispecific protein, and then added with 1:8000 diluted HRP conjugated goat anti-human IgG (H + L) (Cat. No. Ab6858, Abcam, USA) at 100 µL/well, and incubated at 37°C for 0.5 hours. The plate was washed three times with 1 × PBST and added with TMB substrate (Solarbio, 100 µL/well). After incubation at 37°C in dark for 10 minutes, to each well was finally added with 100 µL 2M sulfuric acid to terminate the reaction. The optical density was determined with Synergy Lx Microplate reader (BioTek Instruments, USA) at 450 nm. EC₅₀ represents the desired concentration of the protein samples when the bispecific protein, positive control or negative control protein bind to 50% VEGFA. All tests were conducted in duplicate.

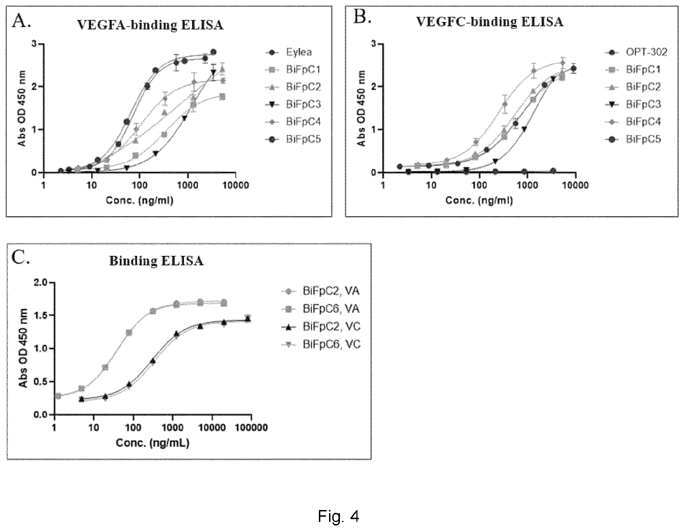

The results are shown in Fig. 4A. As indicated in Fig. 4A, the bispecific protein of the present invention, i.e., BiFpC1, BiFpC2, BiFpC3, BiFpC4 and BiFpC5, can bind to VEGFA in a concentration dependent manner, with a binding effect comparable to that of the positive control Eylea.

### 4.2 Test for the binding of bispecific proteins to VEGFC

A 96-well plate was coated with 0.2 µg/mL VEGFC (Cat. No.: Z03286-10µg, Genscript, China) in a phosphate buffer solution (1× PBS) at 50 µL/well, and then sealed at 4°C overnight for coating. Each well was washed twice with a washing buffer solution 1× PBST (8.01g NaCl, 0.2g KCl, 1.42g Na₂HPO₄, 0.27g KH₂PO₄, 0.05% Tween-20, pH7.4), added with PBST containing 1% BSA (BIOFROXX) at 200 µL/well for blocking. After the plate was sealed and incubated at 37°C for 2 hours, each well was washed twice with 1× PBST to remove the blocking agent. And then added with the bispecific protein of the present invention, i.e., BiFpC1, BiFpC2, BiFpC3, BiFpC4 and BiFpC5, as well as positive control OPT-302, as 4-fold series dilution (0.04-40 nM, diluted in 1× PBS), at 100 µL/well. After incubation at 37°C for 1 hour, each well was washed three times with 1× PBST to remove any unbound bispecific protein, and then added with 1:8000 diluted HRP conjugated goat anti-human IgG (H + L) (Cat. No. Ab6858, Abcam, USA) at 100 µL/well, and incubated at 37°C for 0.5 hours. The plate was washed three times with 1× PBST and added with TMB substrate (Solarbio, 100 µL/well). After incubation at 37°C in dark for 10 minutes, to each well was finally added with 100 µL 2M sulfuric acid to terminate the reaction. The optical density was immediately determined with Synergy Lx Microplate reader (BioTek Instruments, USA) set at 450 nm. EC₅₀ represents the desired concentration of the protein samples when the bispecific proteins, positive control or negative control protein bind to 50% VEGFC. All tests were conducted in duplicate.

The results are showed in Fig. 4B. As indicated in Fig. 4B, BiFpC1, BiFpC2, BiFpC3, and BiFpC4 of the present invention can bind to VEGFC in a concentration dependent manner, with a binding comparable to that of the positive control OPT-302. Bispecific protein BiFpC5 essentially does not bind to VEGFC.

In addition, it can be seen from Figs. 4A-B that there is significant difference in binding to the two targeted sites among bispecific proteins with different kinds of structure. As to the target VEGFA, although BiFpC1, BiFpC 2 and BiFpC3 share the same binding domain which is same as that of Eylea, they have different binding affinities to VEGFA; meanwhile, BiFpC4 and BiFpC5 share another same binding domain but differ from each other in binding to VEGFA. Moreover, literatures shows that the binding domain of BiFpC1, BiFpC2 and BiFpC3 is far superior to that of BiFpC4 and BiFpC5 in binding to VEGFA, but the data from the experiment of this example suggests that BiFpC4 and BiFpC5 instead have higher binding affinity. As to the target VEGFC, although BiFpC1, BiFpC2, BiFpC3, BiFpC4, BiFpC5 share the same VEGFC-binding domain with control molecule OPT302, there is still a significant difference in binding to the target, especially, BiFpC5 completely loses VEGFC-binding affinity. The above results indicate that, due to the differenence in the overall structure, different molecules will exhibit completely different target-binding abilities even if they have the same target-binding domain, and the target-binding abilities may be increased, decreased, or even lost.

### 4.3 Test for the binding of BiFpC2 and BiFpC6 to VEGFA or VEGFC

This example compares the binding of BiFpC2 and BiFpC6 to antigen VEGFA or VEGFC.

A 96-well plate was coated with 0.5 µg/mL VEGFA (Cat. No.: Z03073-10µg, Genscript, China) in a carbonate buffer solution (1× CBS) at 100 µL/well, and then sealed at 4°C overnight for coating. Each well was washed twice with a washing buffer solution 1× PBST (8.01g NaCl, 0.2g KCl, 1.42g Na₂HPO₄, 0.27g KH₂PO₄, 0.05% Tween-20, pH7.4), added with PBST containing 1% BSA (BIOFROXX) at 200 µL/well for blocking. After the plate was sealed and incubated at 37°C for 2 hours, each well was washed twice with 1× PBST to remove the blocking agent, and then added with 4-fold series dilution of BiFpC2 or BiFpC6 (diluted in a phosphate buffer solution 1× PBS). After incubation at 37°C for 1 hour, each well was washed three times with 1× PBST to remove any unbound bispecific protein, and then added with 1:10000 diluted HRP conjugated goat anti-human IgG (H + L) (Cat. No. Ab97225, Abcam, USA) at 100 µL/well, and incubated at 37°C for 0.5 hours. The plate was washed three times with 1× PBST and added with TMB substrate (Tiangen, 100 µL/well). After incubation at 37°C in dark for 10 minutes, to each well was finally added with 50 µL 2M sulfuric acid. The optical density was immediately determined with Synergy Lx Microplate reader (BioTek Instruments, USA) at 450 nm. EC₅₀ represents the desired concentration of the protein samples when the bispecific protein binds to 50% VEGFA. All tests were conducted in duplicate.

A 96-well plate was coated with 2.0 µg/mL VEGFC (Cat. No.: Z03286-10µg, Genscript, China) in a carbonate buffer solution (1× CBS) at 100 µL/well, and then sealed at 4°C overnight for coating. Each well was washed twice with a washing buffer solution 1× PBST (8.01g NaCl, 0.2g KCl, 1.42g Na₂HPO₄, 0.27g KH₂PO₄, 0.05% Tween-20, pH7.4), added with PBST containing 1% BSA (BIOFROXX) at 200 µL/well for blocking. After the plate was sealed and incubated at 37°C for 2 hours, each well was washed twice with 1× PBST to remove the blocking agent, and then added with 4-fold series dilution of bispecific protein BiFpC2 or bispecific protein BiFpC6 (diluted in a phosphate buffer solution 1× PBS). After incubation at 37°C for 1 hour, each well was washed three times with 1× PBST to remove any unbound bispecific protein, and then added with 1:10000 diluted HRP conjugated goat anti-human IgG (H + L) (Cat. No. Ab97225, Abcam, USA) at 100 µL/well, and incubated at 37°C for 0.5 hours. The plate was washed three times with 1× PBST and added with TMB substrate (Tiangen, 100 µL/well). After incubation at 37°C in dark for 10 minutes, to each well was finally added with 50 µL 2M sulfuric acid to terminate the reaction. The optical density was immediately determined with Synergy Lx Microplate reader (BioTek Instruments, USA) at 450 nm. EC₅₀ represents the desired concentration of the protein samples when the bispecific protein binds to 50% VEGFC. All tests were conducted in duplicate.

As shown in Fig. 4C, the bispecific protein of the present invention, i.e., BiFpC2 and BiFpC6, can bind to VEGFA or VEGFC in a concentration dependent manner, and their binding to VEGFA and VEGFC are comparable.

### Example 5: Test for the binding of bispecific proteins to VEGFA and VEGFC

As mentioned earlier, VEGFA can bind to VEGFR1 and VEGFR2, while VEGFC can bind to VEGFR2 and VEGFR3. In order to demonstrate that the candidate bispecific protein constructed in the present invention can specifically bind to VEGFA and VEGFC, this example further tested the binding affinity of the candidate bispecific protein through surface plasmon resonance (SPR). After taking the above data into comprehensive consideration, the structures with ideal data, i.e., BiFpC1, BiFpC2, BiFpC4, and BiFpC6, were selected for further evaluating target affinity using Biacore.

### 5.1 Test for the binding of bispecific proteins to VEGFA

Surface plasmon resonance analysis was performed on Biocore S200 or Biocore 8000 instrument (GE Healthcare), using HEPES-EP+ (GE Healthcare, 10 mM Hepes pH 7.4 + 150 mM NaCl + 3 mM EDTA + 0.05% (v/v) surfactant P20) as a running buffer, at 25°C, to determine the binding affinity of candidate bispecific proteins, i.e., BiFpC1, BiFpC2, BiFpC4, and BiFpC6, to human VEGFA (Cat. No.: Z03073-10 µg, Genscript, China). 10 µg/mL of each of four candidate proteins and a positive control specifically binding to VEGFA (Eylea, Bayer, Germany) were respectively captured onto protein A chip (Cat. No.: 29157555, GE Healthcare) through coupling with protein A. In addition, human VEGFA was diluted in a running buffer to produce human VEGFA solutions at concentrations of 10, 5, 2.5, 1.25, 0.63, 0.31, 0.16, 0.08, and 0 (blank) nM.

Each analysis cycle comprises: (1) capturing the candidate proteins mentioned above on flow pool 2 (Fc2); (2) injecting the VEGFA solution onto flow cells 1 and 2 (Fc1 and Fc2) at a rate of 30 µL/minute (for 120 seconds); (3) allowing 1× HBS-EP + buffer to flow at 30 µL/minute through the chip for 360 seconds to monitor the dissociation phase; (4) injecting a 10 mM glycine hydrochloride buffer, pH 1.5, to allow surface regeneration of the chip. The data was processed by Biacore^{™} Insight Evaluation software, using standard double reference and fitting with a 1:1 binding model, to determine the binding rate (kₐ) and dissociation rate (k_{d}). Equilibrium dissociation constant (K_{D}) is calculated according to the equation K_{D}=k_{d}/kₐ, and the results are shown in Table 3.

**Table 3: Binding affinity of candidate bispecific proteins to VEGFA**

| bispecific protein | kₐ (M⁻¹s⁻¹) | k_{d} (s⁻¹) | K_{D} (M) |
|---|---|---|---|
| Eylea | 2.20E + 07 | 7.96E-04 | **3.61E-11** |
| BiFpC1 | 2.61E + 07 | 4.99E-04 | **1.91E-11** |
| BiFpC2 | 1.51E + 07 | 8.56E-04 | **5.67E-11** |
| BiFpC4 | 2.35E + 07 | 2.39E-04 | **1.02E-11** |
| BiFpC6 | 8.06E + 06 | 2.36E-04 | **2.93E-11** |

From the results in Table 3, it can be seen that BiFpC1, BiFpC2, BiFpC4, and BiFpC6 of the present invention all have a high binding affinity with VEGFA, with similar K_{D} values to Eylea, at a magnitude up to 10⁻¹¹ M.

### 5.2 Test for the binding of bispecific proteins to VEGFC

Surface plasmon resonance analysis was performed on Biocore S200 or Biocore 8000 instrument (GE Healthcare) with an analysis temperature of 25 °C to determine the binding affinity of the bispecific proteins of the present invention to human VEGFC, using HEPES-EP+ (GE Healthcare, 10 mM Hepes pH 7.4 + 150 mM NaCl + 3 mM EDTA + 0.05% (v/v) surfactant P20) as a running buffer.

Each of bispecific proteins BiFpC1, BiFpC2, BiFpC4, BiFpC6 as well as positive control OPT-302 specifically recognizing VEGFC was respectively captured onto protein A chip (Cat. No.: 29157555, GE Healthcare) through coupling with protein A with a fixed concentration 10 µg/mL. Human VEGFC (Cat. No.: Z03286-10µg, Genscript, China) solutions with concentrations of 10, 5, 2.5, 1.25, 0.63, 0.31, 0.16, 0.08, and 0 (blank) nM were produced by diluted into a running buffer. Each analysis cycle comprises: (1)capturing the bispecific protein on flow pool 2 (Fc2), (2) injecting the VEGFC solution onto flow cells 1 and 2 (Fc1 and Fc2) at a rate of 30 µL/minute (for 120 seconds), (3) allowing 1× HBS-EP + buffer to flow at 30 µL/minute through the chip for 360 seconds to monitor the dissociation phase, (4) injecting a 10 mM glycine hydrochloride buffer, pH 1.5, to allow surface regeneration of the chip. The data was processed by Biacore^{™} Insight Evaluation software, using standard double reference and fitting with a 1:1 binding model, to determine the binding rate (kₐ) and dissociation rate (k_{d}). Equilibrium dissociation constant(K_{D}) is calculated according to the equation K_{D}=k_{d}/kₐ, and the results are shown in Table 4.

**Table 4: Binding affinity of candidate bispecific proteins to VEGFC**

| bispecific protein | kₐ(M⁻¹s⁻¹) | k_{d}(s⁻¹) | K_{D}(M) |
|---|---|---|---|
| OPT-302 | 5.87E + 06 | 3.30E-03 | **5.62E-10** |
| BiFpC1 | 1.08E + 07 | 4.03E-03 | **3.72E-10** |
| BiFpC2 | 2.67E + 06 | 2.47E-04 | **9.23E-11** |
| BiFpC4 | 1.91E + 07 | 1.91E-03 | **1.00E-10** |
| BiFpC6 | 3.17E + 06 | 2.00E-04 | **6.33E-11** |

From the results in Table 4, it can be seen that BiFpC1, BiFpC2, BiFpC4, and BiFpC6 of the present invention all have a high binding affinity to VEGFC, with K_{D} value of up to 10⁻¹⁰ M magnitude, wherein BiFpC2 and BiFpC6 have a K_{D} value even up to 10⁻¹¹ M magnitude, far exceeding that of OPT-302(at 10⁻¹⁰ M magnitude), demonstrating a higher affinity for VEGFC.

From the above results, it can be seen that all four structures tested exhibit strong VEGFA affinity, but there is a significant difference in their affinity for VEGFC, wherein BiFpC2 and BiFpC6 have extremely strong VEGFC affinity, which is about 10 times that of the control molecule OPT-302. This again demonstrates that, even if the same structural domain is used, the difference in molecular structure will greatly affect the binding ability of molecules to target molecules.

### Example 6: Verification of the simultaneous binding of the bispecific protein of the present invention to VEGFA and VEGFC

This example tests whether the bispecific protein of the present invention, i.e., BiFpC2 and BiFpC6, can bind to VEGFA and VEGFC simultaneously.

Surface plasmon resonance analysis was performed on Biacore 8000 instrument with an analysis temperature of 25 °C, using HEPES-EP+ (GE Healthcare, 10 mM Hepes pH7.4 + 150 mM NaCl + 3 mM EDTA + 0.05%(v/v) surfactant P20) as a running buffer, to determine the binding ability of bispecific proteins of the present invention, i.e., BiFpC2 and BiFpC6, to human VEGFA and/or VEGFC.

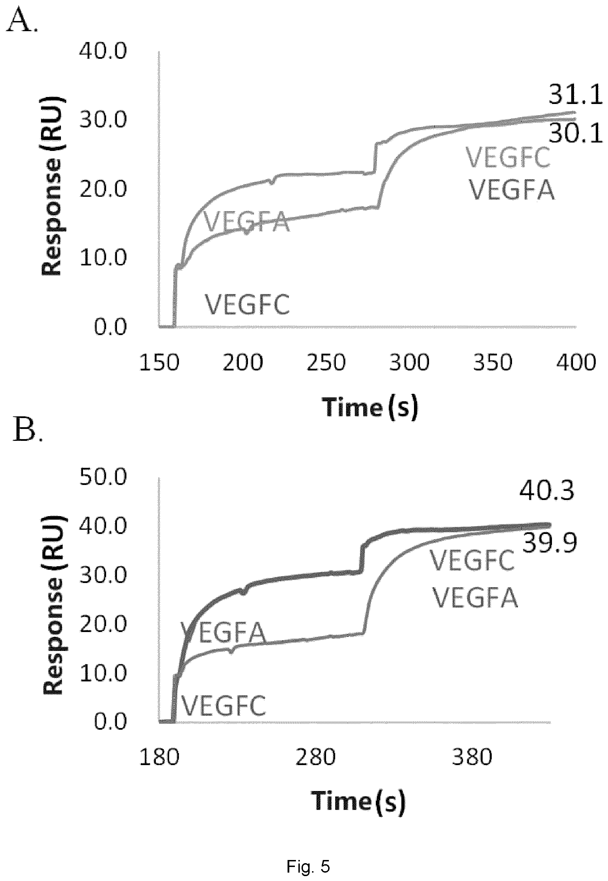

The bispecific proteins, BiFpC2 and BiFpC6, obtained from Example 3, were allowed to pass through Protein A chip at a fixed concentration of 10 µg/mL, so as to be captured on the Protein A chip (Cat. No.: 29157555, GE Healthcare) through coupling with Protein A. An A-B-A continuous injection mode was adopted, and the operation is consisted of: capturing the bispecific protein on flow pool 2 (Fc2) for 30-40 seconds, injecting 20 nM VEGFA or 500 nM VEGFC at 30 µL/ minutes (for 120 seconds) onto flow cell 1 and flow cell 2 (Fc1 and Fc2), and injecting 500 nM VEGFC or 20 nM VEGFA at 30 µL/ minutes (for 120 seconds) onto flow cell 1 and flow cell 2 (Fc1 and Fc2), finally injecting 10 mM glycine hydrochloride buffer (pH 1.5) at 30 µL/ minutes (for 60 seconds) to allow surface regeneration of the chip. The data was processed by Biacore^{™} Insight Evaluation software, using standard double reference and fitting with a 1:1 binding model. The results are shown in Fig. 5.

As shown in Fig. 5A, the ability of the bispecific protein BiFpC2 of the present invention for binding to both human VEGFA and human VEGFC simultaneously is conformed through the increase in resonance units (initial 22.3 RU from VEGFA, followed by an additional 8.8 RU from human VEGFC) from two ligands bounded to the bispecific protein and through the increase in resonance units (initial 17.3 RU from VEGFC, followed by an additional 12.8 RU from human VEGFA) in another injection method. Moreover, after the binding of bispecific protein BiFpC2 to the two ligands in different injection orders, the cumulative resonance units were 31.1 RU and 30.1 RU, respectively, and there was no significant difference between them.

As shown in Fig. 5B, the ability of another bispecific protein BiFpC6 of the present invention for binding to both human VEGFA and human VEGFC simultaneously is conformed through the increase in resonance units (initial 30.6 RU from VEGFA, followed by an additional 9.8 RU from human VEGFC) from two ligands bounded to the bispecific protein and through the increase in resonance units (initial 17.6 RU from VEGFC, followed by an additional 22.0 RU from human VEGFA) in another injection method. Moreover, after the binding of bispecific protein BiFpC6 to the two ligands in different injection orders, the cumulative resonance units were 40.3 RU and 39.9 RU, respectively, and there was no significant difference between them.

### Example 7: Test for stability of the bispecific protein of the present invention at different temperatures

The bispecific proteins BiFpC2 and BiFpC6 obtained in Example 3 were subjected to affinity chromatography, and then put into a buffer system of 20 mM citric acid-sodium citrate, pH 4.8. The samples were subjected to the placement and collection according to the following protocol for stability testing.

The samples were placed: at low temperature (4°C) for respectively 1, 3, 6, 15, 30, 60, and 90 days; at room temperature (25°C) for respectively 3, 6, 15, and 30 days; and at high temperature (40°C) for respectively 1, 3, and 6 days. The samples were respectively sampled at time zero and various sampling time point, and the collected bispecific protein samples were subjected to size-exclusion chromatography (SEC)HPLC for analysis based on the percentage of molecular weight of a target protein (main peak%). Specifically, the sample to be analyzed was diluted to 1.0 mg/mL, injected at 10 µL (10 µg) onto a molecular sieve column (Sepax Zenix-C SEC-300) to be analysed. The test was performed at the following conditions: a flow rate of 0.65 mL/ minutes, a column temperature of 35°C, detection wavelength of 214 nm, and a mobile phase of 200 mM phosphate buffer solution + 300 mM NaCl, pH 7.0. The bispecific protein peak (main peak), aggregate peak and fragment peak were determined by integration of appropriate peaks. The results were obtained by using ChemStation to analyze chromatograph, and using the ratio of AUC of the eluted peaks between the aggregate peaks and fragment peaks to total AUC to calculate the main peak (%). The results are shown in Table 5 below.

**Table 5: Stability of bispecific proteins BiFpC2 and BiFpC6 at different temperatures.**

| Sample | | BiFpC2 | | | BiFpC6 | | |
|---|---|---|---|---|---|---|---|
| Conditions | days | Aggregate peak(%) | main peak (%) | fragment peak(%) | Aggregate peak(%) | main peak (%) | fragment peak(%) |
| untreated | 0 | 12.6 | 84.2 | 3.2 | 12.5 | 87.0 | 0.5 |
| high temperature | 1 | 24.8 | 72.6 | 2.7 | 19.0 | 79.1 | 1.9 |
| | 3 | 43.1 | 53.4 | 3.5 | 30.6 | 66.4 | 3.0 |
| | 6 | 60.9 | 35.3 | 3.8 | 46.5 | 49.7 | 3.8 |
| room temperature | 3 | 12.2 | 85.5 | 2.2 | 12.4 | 86.7 | 1.0 |
| | 6 | 12.7 | 84.5 | 2.7 | 12.7 | 86.6 | 0.7 |
| | 15 | 14.2 | 81.1 | 4.7 | 11.9 | 85.5 | 2.6 |
| | 30 | 13.3 | 81.1 | 5.5 | 11.2 | 84.6 | 4.2 |
| low temperature | 1 | 13.2 | 84.0 | 2.7 | 12.2 | 85.9 | 1.9 |
| | 3 | 12.0 | 85.9 | 2.1 | 11.9 | 87.8 | 0.3 |
| | 6 | 11.8 | 85.9 | 2.3 | 12.0 | 86.9 | 1.2 |
| | 15 | 10.4 | 85.6 | 4.1 | 10.5 | 88.7 | 0.8 |
| | 30 | 10.3 | 85.7 | 4.0 | 10.5 | 89.5 | 0.0 |
| | 60 | 12.1 | 84.7 | 3.2 | 12.0 | 85.4 | 2.6 |
| | 90 | 12.5 | 84.9 | 2.6 | 11.9 | 85.8 | 2.3 |

The data from the above table indicates that the bispecific proteins BiFpC2 and BiFpC6 are stable under room temperature and low temperature conditions. At room temperature of 25 °C for 30 days without adding any other excipients, the main peak of bispecific protein BiFpC2 changed from 84.2% to 81.1%, and the main peak of bispecific protein BiFpC6 changed from 87.0% to 84.6%. At low temperature of 4°C for 90 days, the main peak of bispecific protein BiFpC2 changed from 84.2% to 84.9%, and the main peak of bispecific protein BiFpC6 changed from 87.0% to 85.8%. In contrast, bispecific proteins BiFpC2 and BiFpC6 are less stable under high temperature conditions. At 40°C for 6 days, the main peak of bispecific protein BiFpC2 changed from 84.2% to 35.3%, and the main peak of bispecific protein BiFpC6 changed from 87.0% to 49.7%. Therefore, bispecific proteins should be kept from high temperature environments during the manufacturing process. BiFpC6 shows a better stability than BiFpC2.

### Example 8: Inhibition of the bispecific protein of the present invention on HUVEC cell proliferation induced by VEGFA or VEGFC

HUVEC cell refers to human umbilical vein endothelial cell, which is a most commonly used cell line for *in vitro* detection of drugs related to the VEGF/VEGFR signaling pathway. The HUVEC cells used in this example are commercially availed from Sciencell Corporation (Cat. No. #8000).

### 8.1 On HUVEC cell proliferation induced by VEGFA

VEGFA refers to an endothelial growth factor, which initiates relevant intracellular signaling pathways by binding to cell membrane surface receptors VEGFR1 and VEGFR2, and participates in physiological and pathological processes such as cell proliferation and angiogenesis.

A dose range of bispecific protein from 0.1 ng/mL to 1 µg/mL was evaluated. The bispecific protein BiFpC2 of each test concentration was added at 50 µL to a well containing 50 µL 10 ng/mL (final concentration) VEGFA. The test was conducted in duplicate. Commercially available VEGFA-neutralizing protein Eylea was used as positive control, and the well added with only test culture mediumwas used as solvent blank control. The control Eylea was tested at the same mass concentration range as bispecific protein BiFpC2. The 96-well plate containing VEGFA/protein mixture was incubated at 37°C, 95% relative humidity, 5% CO₂ for 60 minutes.

HUVEC cells in ECM complete medium (containing 5% FBS, penicillin G (1×) and streptomycin (1×) and endothelial cell growth additive ECGS (1×)) were incubated in an incubator at 37°C, 5% CO₂ for 24 hours, then rinsed with 1× DPBS (free of Ca²⁺, Mg²⁺), digested with 1 mL 0.6% trypsin/EDTA, to detach the cells from the bottom of the culture bottle, and then added with 9 mL ECM complete medium to terminate the digestion reaction. After that, the cells were centrifuged at 1000 ×g for 5 minutes at room temperature. The cells pellet was resuspended in test culture medium (ECM test culture medium containing 0.5% FBS). The density of the cell was measured with an automatic cell counter (Countstar, Cat. No.: IC1000, Motic). After adjusting the density, the cells were added (in 100 µL) at 5000/well into the center wells of a 96-well plate, and DPBS was added into the surrounding wells to prevent medium evaporation during the cultivation process. The 96-well plate was placed in a tissue incubator (37°C, 95% relative humidity, 5% CO₂) overnight. After 24 hours, the HUVEC cells cultured in the 96-well plate were added with the protein/VEGFA mixture (100 µL), and incubated for 72 hours.

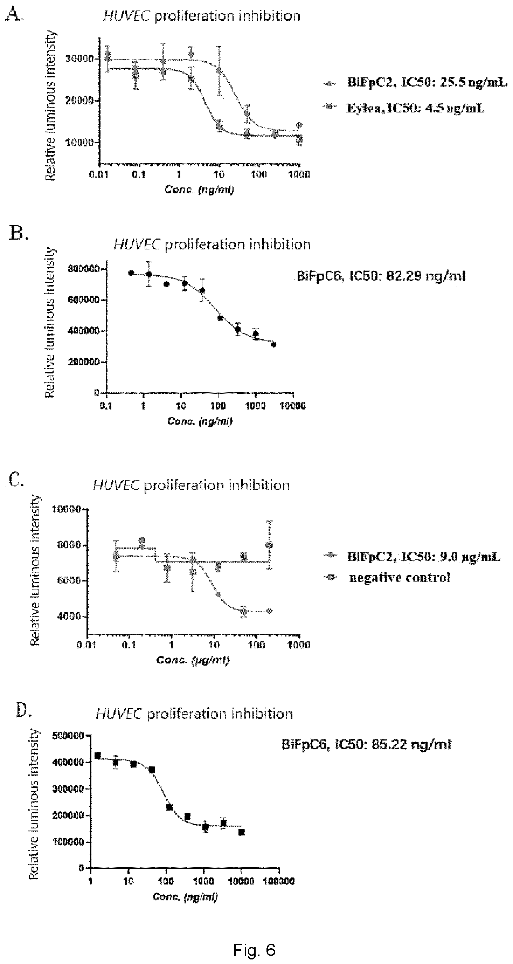

When the reaction was completed, the HUVEC cells viability was tested with CellTiter-Glo^{®} cell viability test kit (Promega, USA) according to the product manual. In brief, the test kit was equilibrated to room temperature, then CellTiter-Glo^{®} buffer solution was injected into CellTiter-Glo^{®} Substrate with a syringe, mixed homogeneously, and added at 100 µL/well into cells of the 96-well plate from which 100 µL/well culture medium has been removed. After reacting at room temperature for 10 minutes, chemiluminescence was determined with microplate reader Synergy Lx (BioTek Instruments, USA). The results are shown in Fig. 6A. The relative luminescence values shown in Fig. 6A are calculated using the 4-parameter inhibition curve fitting (GraphPad Prism, version 5.0) of the data, wherein the concentration of bispecific protein BiFpC2 or Eylea is used as IC₅₀ when 50% of the VEGFA induced response is inhibited. As shown in Fig. 6A, the bispecific protein BiFpC2 of the present invention inhibits HUVEC cell proliferation induced by VEGFA in a concentration dependent manner, and thereby demonstrating the ability of the bispecific protein BiFpC2 of the present invention to effectively neutralize VEGFA.

Through a process similar to that for BiFpC2, the inhibitory effect of BiFpC6 on HUVEC cell proliferation induced by VEGFA is verified. The testing procedure is as follows:

A dose range of the bispecific protein from 0.46 ng/mL to 3 µg/mL (final concentration) was evaluated. Each test concentration of the bispecific protein BiFpC6 was added at 45 µL to a well containing 45 µL 45 ng/mL VEGFA. The test was conducted in duplicate. The wells added with only test culture medium were used as solvent blank control. The 96-well plate containing VEGFA/protein mixture was incubated at 37°C, 95% relative humidity, 5% CO₂ for 60 minutes.

HUVEC cells were cultured in a T75 cell culture bottle containing ECM complete medium (containing 5% FBS, penicillin and streptomycin (1×) and endothelial cell growth additive ECGS (1×)) at 37°C and 5% CO₂ in an incubator. 24 hours before adding samples to be tested in the proliferation experiment, the cells were rinsed with 1 × PBS, digested with 3 mL 0.25% trypsin/EDTA to detach the cells from the bottom of the culture bottle, and then added with 7 mL ECM complete medium to terminate the digestion reaction. After that, the cells were centrifuged at 150 ×g for 5 minutes at room temperature. The cells pellet was resuspended with test culture medium (ECM culture medium containing 0.5% FBS). The density of the cell was measured with an automatic cell counter (Countstar, Cat. No.: IC1000, Motic). After adjusting the density, the cells were added (in 100 µL) at 5000/well into the center wells of a 96-well plate, and sterilized water was added into the surrounding wells to prevent medium evaporation during the cultivation process. The 96-well plate was placed in a tissue incubator (37°C, 95% relative humidity, 5% CO₂) overnight. After 24 hours, the HUVEC cells cultured in the 96-well plate were added with the protein/VEGFA mixture (80 µL), and incubated for 72 hours, wherein the final concentration of VEGFA was 10 ng/mL.

When the reaction was completed, HUVEC cells viability was tested with CellTiterGlo cell viability testing reagent. In brief, the test kit was equilibrated to room temperature, then each well was added with 90 µL testing reagent, shaken on a shaker for 2 minutes. After reacting at room temperature for 10 minutes, chemiluminescence was determined with Fluoroskan^{™} microplate reader (ThermoFisher). The results are shown in Fig. 6B. Fig. 6B shows the inhibition curve generated using 4-parammeter curve fitting (GraphPad Prism, version 8.0), wherein, the concentration of bispecific protein BiFpC6 is used as IC₅₀ when 50% of the VEGFA induced response is inhibited. As shown in Fig. 6B, the bispecific protein of the present invention BiFpC6 inhibits HUVEC cell proliferation induced by VEGFA in a concentration dependent manner, and thereby demonstrating the ability of the bispecific protein BiFpC6 of the present invention to effectively neutralize VEGFA.

### 8.2 On HUVEC cell proliferation induced by VEGFC

VEGFC refers to an endothelial growth factor, which initiates relevant intracellular signaling pathways by binding to cell membrane surface receptors VEGFR2 and VEGFR3, and participates in physiological and pathological processes such as cell proliferation and angiogenesis.

A dose range of the bispecific protein from 0.01µg/mL to 200 µg/mL was evaluated. Each test concentration of the bispecific protein BiFpC2 was added at 50 µL to a well containing 50 µL 100 ng/mL (final concentration) VEGFC. The test was conducted in duplicate. Monoclonal antibody FTL001 (Sound Biopharma, China) was used in the test as negative control, and the well added with only test culture medium wasused as solvent blank control. Control monoclonal antibody was tested at the same mass concentration range as the bispecific protein. The 96-well plate containing VEGFC/ protein mixture was incubated at 37°C, 95% relative humidity, 5% CO₂ for 60 minutes.

HUVEC cells were cultured as usual in complete medium (endothelial cell culture medium ECM, containing 5% FBS, penicillin G (1×) and streptomycin (1×) and endothelial cell growth additive ECGS (1×)). After 24 hours, the cells were rinsed with 1 × DPBS (free of Ca²⁺, Mg²⁺), digested with 1 mL 0.6% trypsin/EDTA to detach the cells from the bottom of the culture bottle, and added with 9 mL complete medium to terminate the digestion reaction. After that, cells were low-speed centrifuged at 1000 ×g for 5 minutes at room temperature. The cells pellet was resuspended in test culture medium (ECM test culture medium containing 0.5% FBS). The density of the cell is measured with an automatic cell counter (Countstar, Cat. No.: IC1000, Motic). After adjusting the density, the cells were added (in 100 µL) at 5000/well into center wells of a 96-well plate, and DPBS was added into the surrounding wells to prevent medium evaporation during the cultivation process. The 96-well plate was placed in a tissue incubator (37°C, 95% relative humidity, 5% CO₂) overnight. After 24 hours, the HUVEC cells were added with the bispecific protein (or monoclonal antibody)/VEGFC mixture (100 µL) , and incubated for 72 hours.

When the reaction was completed, according to CellTiter-Glo^{®}(Promega, USA) manual, the test kit was equilibrated to room temperature to be used. Before using, CellTiter-Glo^{®} Buffer was injected into CellTiter-Glo^{®}Substrate with a syringe and mixed homogeneously. 100 µL culture medium was removed, and then 100 µL mixed test solution was added. After reacting at room temperature for 10 minutes, chemiluminescence was determined using microplate reader Synergy Lx (BioTek Instruments, USA). The results are shown in Fig. 6C.

The relative luminescence value shown in Fig. 6C is calculated using the 4-parameter inhibition curve fitting (GraphPad Prism, version 5.0) of the data. The concentration of bispecific protein BiFpC2 or negative control is used as IC₅₀ when 50%of response induced by VEGFC is suppressed. As can be seen from Fig. 6C, the bispecific protein BiFpC2 inhibits HUVEC cell proliferation induced by VEGFC in a concentration dependent manner, demonstrating the ability of the bispecific protein BiFpC2 of the present invention to effectively neutralize VEGFC.

Through a process similar to that for BiFpC2, the inhibitory effect of BiFpC6 on HUVEC cell proliferation induced by VEGFC is verified. The testing procedure is as follows:
A dose range of bispecific protein from 1.5 ng/mL to 10 µg/mL (final concentration) was evaluated. Each test concentration of the bispecific protein BiFpC6 was added at 45 µL to a well containing 45 µL 225 ng/mL VEGFC. The test was conducted in duplicate. The wells added with only test culture medium were used as solvent blank control. The 96-well plate containing VEGFC/protein mixture was incubated at 37°C, 95% relative humidity, 5% CO₂ for 60 minutes.

HUVEC cells were cultured in a T75 cell culture bottle containing ECM complete medium (containing 5% FBS, penicillin and streptomycin (1×) and endothelial cell growth additive ECGS (1×)) at 37°Cand 5% CO₂ in an incubator. 24 hours before adding sample to be tested into the proliferation experiment, the cells were rinsed with 1× PBS, digested with 3 mL 0.25% trypsin/EDTA to detach the cells from the bottom of the culture bottle, and then added with 7 mL ECM complete medium to terminate the digestion reaction. After that, the cells were centrifuged at 150 ×g for 5 minutes at room temperature. The cells pellet is resuspended in test culture medium (ECM culture medium containing 0.5% FBS). The density of the cell was measured with an automatic cell counter (Countstar, Cat. No.: IC1000, Motic). After adjusting the density, the cells were added (in100 µL) at 5000/well into the center wells of a 96-well plate, and sterilized water was added into the surrounding wells to prevent medium evaporation during the cultivation process. The 96-well plate was placed in a tissue incubator (37°C, 95% relative humidity, 5% CO₂) overnight. After 24 hours, the HUVEC cells cultured in the 96-well plate were added with protein/VEGFC mixture (80 µL), and incubated for 72 hours, wherein the final concentration of VEGFC was 50 ng/mL.

When the reaction was completed, HUVEC cells viability was tested with CellTiterGlo cell viability testing reagent. In brief, the test kit was equilibrated to room temperature, then each well was added with 90 µL testing reagent, and shaken on a shaker for 2 minutes. After reacting at room temperature for 10 minutes, chemiluminescence was determined with Fluoroskan^{™} microplate reader (ThermoFisher). The results are shown in Fig. 6D. Fig. 6D shows the inhibition curve generated using 4-parameter curve fitting (GraphPad Prism, version 8.0), wherein the concentration of bispecific protein BiFpC6 is used as IC₅₀ when 50% of VEGFC induced response is inhibited . As shown in Fig. 6D, the bispecific protein BiFpC6 of the present invention inhibits HUVEC cell proliferation induced by VEGFC in a concentration dependent manner, thereby demonstrating the ability of the bispecific protein BiFpC6 of the present invention to effectively neutralize VEGFC.

### Example 9: Determination of competitive binding of bispecific proteins of the present invention to VEGFA and VEGFC

### 9.1 Competitive binding to VEGFA

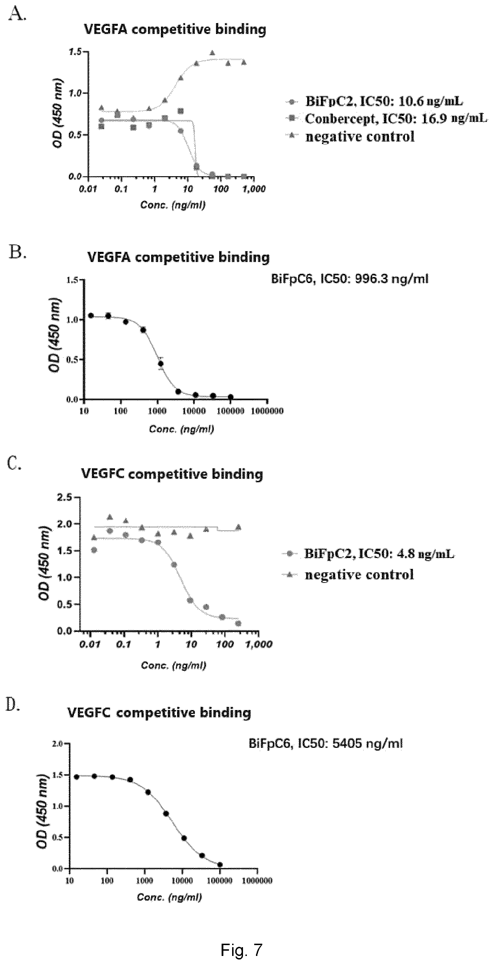

A 96-well plate was coated with 1.0 µg/mL Eylea (Bayer, Germany) in 1× PBS (8.01g NaCl, 0.2g KCl, 1.42g Na₂HPO₄, 0.27g KH₂PO₄, 0.05% Tween-20, pH7.4) at 100 µL/well, and then sealed at 4°C overnight for coating. Each well was washed twice with 1× PBST (1× PBS, 0.05% Tween-20, pH7.4) and added with PBST containing 1% BSA (BIOFROXX) at 200 µL/well for blocking. The plate was sealed and incubated at 37°C for 2 hours, then each well was washed twice with 1× PBST to remove the blocking agent. The bispecific protein BiFpC2 of the present invention, positive control Conbercept eye injection (KANGHONG Pharmaceutical, China) and negative control human IgG1, kappa Isotype Control (Cat. No. HG1K, Sino Biological, China), respectively as 3x series dilutions (in 1× PBS), were pre-incubated with 5 nM VEGFA (Cat. No. Z03073, Genscript, China) dimer at 37°C for 1 hour. When the pre-incubation was completed, the co-incubated mixture was added at 100 µL/well to each well, and incubated at 37°C for 1 hour. Each well was washed three times with 1× PBST, and then added with 1:75000 diluted VEGFA rabbit monoclonal antibody (Cat. No. 11066-R105, Sino Biological, China) at 100 µL/well and incubated at 37°C for 0.5 hours. Each well was washed three times with 1× PBST, and then added with 1:75000 diluted goat anti-rabbit IgG (H + L) polyclonal antibody (Cat. No. 511203, ZEN BIO, China) at 100 µL/well. The plate was washed three times with 1× PBST, added with TMB substrate (Cat. No. PR1200, Solarbio, 100 µL/well), then incubated at 37°C in dark for 10 minutes, and finally added with 50 µL 2M sulfuric acid to each well to terminate the reaction. Optical density was detected at 450 nm with microplate reader Synergy Lx (BioTek Instruments, USA). The test was conducted in duplicate. The results are shown in Fig. 7A, wherein IC₅₀ denotes the concentration of the bispecific protein or positive control when 50% VEGFA is competitively bound by bispecific protein or positive control. As shown in Fig. 7A, the bispecific protein BiFpC2 of the present invention competitively binds to VEGFA with Eylea in a concentration dependent manner, and its competitive binding activity is comparable to that of the positive control Conbercept, wherein bispecific protein BiFpC2 has an IC₅₀ of 10.6 nM, positive control has an IC₅₀ of 16.9 nM, and negative control has no competitive binding activity.

Through a process similar to that for BiFpC2, the competitive binding of BiFpC6 to VEGFA is verified. The testing procedure is as follows:
A 96-well plate was coated with 0.5 µg/mL VEGFA165 (Cat. No. Z03073-50, GenScript, China) in a carbonate coated buffer solution (sodium carbonate 1.59 g, sodium bicarbonate 2.93 g, ultrapure water 1000 mL, pH9.6) at 100 µL/well, and then sealed at 4°C overnight for coating. Each well was washed twice with 1×PBST (1×PBS, 0.05% Tween-20, pH7.4), added with PBST containing 1% BSA (Cat. No. A8020, Solarbio, China) at 260 µL/well for blocking. The plate was sealed and incubated at 37°C for 1.5 hours, then washed twice with 1×PBST to remove the blocking agent. The bispecific protein BiFpC6 of the present invention as 3x series dilutions (in 1×PBST + 0.1%BSA) was well mixed with 0.5 µg/mL Human VEGFR2 (Cat. No. KDR-H5227, AcroBio systems, China) at 1:1 ratio, and added at 100 µL/well to corresponding wells of the assay plate and incubated at 37°C for 1 hour. Each well was washed three times with 1× PBST, added at 100 µL/well to 1:8000 diluted HRP-Anti 6*His antibody (Cat. No. HRP-66005, Proteintech, China), and then incubated at 37°C for 45 minutes. The assay plate was washed three times with 1× PBST, added with TMB substrate (Cat. No. PA107-01, TIANGEN BIOTECH (BEIJING) CO.,LTD., China) at 100 µL/well, incubated at 37°C in dark for 10 minutes. Finally, each well was added with 100 µL oxalic acid terminating solution (anhydrous oxalic acid 63.0 g, ultrapure water 1000 mL) to terminate the reaction. Optical density was detected with microplate reader (Beijing Pulang Biotechnology Co., Ltd., China) at 450 nm. The results shown in Fig. 7B suggest that the bispecific protein BiFpC6 of the present invention competitively binds to VEGFA165 with Human VEGFR2 in a concentration dependent manner, wherein bispecific protein BiFpC6 has an IC₅₀ of 996.3 ng/mL.

### 9.2 Competitive binding to VEGFC

A 96-well plate was coated with 1.25 µg/mL VEGFR3-Fc (Cat. No. FL4-H5251, ACRO Biosystems, China) in 1× PBS (8.01g NaCl, 0.2g KCl, 1.42g Na₂HPO₄, 0.27g KH₂PO₄, 0.05% Tween-20, pH7.4) at 50 µL/well, and then sealed at 4°C overnight for coating. Each well was washed twice 1× PBST (1× PBS, 0.05% Tween-20, pH7.4), added with PBST containing 1% BSA (BIOFROXX) at 200 µL/well for blocking. The plate was sealed and incubated at 37°C for 2 hours, and each well was washed twice with 1× PBST. The bispecific protein BiFpC2 and negative control human IgG1, kappa Isotype Control (Cat. No. HG1K, Sino Biological, China), respectively as 3x series dilutions (in 1× PBS), were pre-incubated with 2.5 nM VEGFC-his (Cat. No. VEC-H4225, AcroBio systems, China) dimer at 37°C for 1 hour. When the pre-incubation is completed, the co-incubated mixture is added to each well (100 µL/well), and incubated at 37°C for 1 hour. Each well was washed three times with 1× PBST, and then added with 1:5000 diluted HRP conjugated 6*His, His-Tag monoclonal antibody (Cat. No. 66005, Proteintech, USA) at 100 µL/well, and incubated at 37°C for 0.5 hours. The plate was washed three times with 1× PBST, added with TMB substrate (Cat. No. PR1200, Solarbio, 100 µL/well), incubated at 37°C in dark for 10 minutes, and finally added with 50 µL 2M sulfuric acid to terminate the reaction. Optical density was detected with microplate reader Synergy Lx (BioTek Instruments, USA) at 450 nm. The test was conducted in duplicate. The results are shown in Fig. 7C. As shown in Fig. 7C, the bispecific protein BiFpC2 competitively binds to VEGFC with natural receptor VEGFR3 in a concentration dependent manner, wherein, bispecific protein BiFpC2 has an IC₅₀ of 4.8 nM, while negative control has no competitive binding activity.

Through a process similar to that for BiFpC2, the competitive binding of BiFpC6 to VEGFC is verified. The testing procedure is as follows:
A 96-well plate was coated with 1 µg/mL Human VEGFC Protein (His Tag) (Cat. No. 10542-H08H, Sino Biological Inc. (BEIJING), China) in a carbonate coated buffer solution (sodium carbonate 1.59 g, sodium bicarbonate 2.93 g, ultrapure water 1000mL, pH9.6) at 100 µL/well, and then sealed at 4°C overnight for coating. Each well was washed twice with 1× PBST (1× PBS, 0.05% Tween-20, pH7.4), added with PBST containing 1% BSA (Cat. No. A8020, Solarbio, China) at 260 µL/well for blocking. The assay plate was sealed and incubated at 37°C for 1.5 hours, and each well was washed twice with 1× PBST. The bispecific protein BiFpC6, as 3x series dilutions (in 1×PBST + 0.1%BSA), was well mixed with 0.1 µg/mL Biotinylated Human VEGFR3/FLT4 Protein, His, Avitag^{™} (Cat. No. FL4-H82E1, AcroBio systems, China) at 1:1 ratio, and, added at 100 µL/well to corresponding wells of the assay plate, and incubated at 37°C for 1 hour. Each well was washed three times with 1× PBST, then added with 1:8000 diluted Streptavidin-HRP (SA) (Cat. No. YXXO5701-HRP, AtaGenix Laboratories, Wuhan, China) at 100 µL/well, and incubated at 37°C for 45 minutes. The plate was washed three times with 1× PBST, added with TMB substrate (Cat. No. PA107-01, TIANGEN BIOTECH (BEIJING) CO.,LTD., China) at 100 µL/well, incubated at 37°C in dark for 10 minutes, and finally added with 100 µL oxalic acid terminating solution (anhydrous oxalic acid 63.0 g, ultrapure water 1000mL) to terminate the reaction. Optical density was detected with microplate reader (Beijing Pulang Biotechnology Co., Ltd., China) at 450 nm. The results shown in Fig. 7D suggest that, the bispecific protein BiFpC6 competitively binds to VEGFC with natural receptor VEGFR3 in a concentration dependent manner, wherein the bispecific protein BiFpC6 has an IC₅₀ of 5405ng/mL.

### Example 10: in vivo assay of the bispecific protein of the present invention in Macaca fascicularis nCNV model

Eight **Macaca *fascicularis*** (available from Guangxi Xiongsen Primate Experimental Animal Breeding and Development Co., Ltd.) were used as a wet age-related macular degeneration (neoAMD) model. All animals were recorded as D1 on the day of model establishment. Choroidal neovascularization (CNV) in both eyes of **Macaca *fascicularis*** was induced through fundus laser photocoagulation. At D15, the fundus fluorescence leakage of animals was evaluated by fundus fluorescence angiography (FFA) test, and CNV leakage was graded according to the following criteria:
Grade 1, no high fluorescence spot;
Grade 2, high fluorescence spot, no fluorescence leakage;
Grade 3, high fluorescence spot, mild fluorescence leakage, within the spot edge;
Grade 4, high fluorescence spot, severe fluorescence leakage, beyond the spot edge.

At D16, 5 animals with grade 4 leakage were selected and divided into two groups (based on the spot average leakage area of grade 4 and on the spot ratio of grade 4, so that there is no significant difference between the two groups). One group of three animals were administered with the bispecific protein BiFpC2 of the present invention; another group of 2 animals were administered with positive control Eylea. On the day of grouping, animals were intravitreously injected once in a single eye with 100 µL of 20 µM the bispecific protein BiFpC2 or 43 µM positive control Eylea. During the assay, animals were subjected daily to general clinical observations, and were weighed and subjected to general ophthalmic examination on D1, D15, D18, D22, and D29. Fundus photography (FP), FFA, and optical coherence tomography (OCT) were performed on animals on D1, D15, D22, and D29. The effectiveness was evaluated based on four indicators including, such as, fluorescence leakage area of spots of grade 4 and the thickness of subretinal high reflective membrane (SHRM). The results are as follows:
Fluorescence spot ratio of grade 4: on D22 (1 week after injection) and D29 (2 weeks after injection), compared to those before treatment, animals treated with bispecific protein BiFpC2 and positive control Eylea show a significant decrease in fluorescence spot ratio of grade 4, and there is no statistically significant difference (p>0.05) between the bispecific protein BiFpC2 group and the positive control Eylea group.

Fluorescence spot average leakage area of grade 4: on D15 (before injection), there is no statistically significant difference (p>0.05) in the spot average leakage area of grade 4 between the bispecific protein BiFpC2 group and the positive control Eylea group. on D29, both the bispecific protein BiFpC2 and the positive control Eylea lead to reduced fluorescence leakage area, and there is no statistically significant difference in the average fluorescence leakage area between the two groups (p>0.05).

The reduction and improvement rate of fluorescence spot average leakage area of grade 4: on D22 and D29, there is no statistically significant difference (p>0.05) in the reduction of average fluorescence leakage area reduction between bispecific protein BiFpC2 and the positive control Eylea group. On D29, there is no statistically significant difference (p>0.05) in the improvement rate of average fluorescence leakage area between the bispecific protein BiFpC2 group and the positive control group.

SHRM average thickness: On D15, D22 and D29, there is no statistically significant difference (p > 0.05) in SHRM average thickness between bispecific protein BiFpC2 and the positive control Eylea group.

This example used a **Macaca *fascicularis*** choroidal neovascularization model, with a single intravitreal injection of 100 µL of 20 µM bispecific protein BiFpC2 or 43 µM positive control Eylea. One week (D22) and two weeks (D29) after injection, it was observed that the bispecific protein BiFpC2 leads to a reduced fluorescence leakage area and decreased thickness of subretinal high reflective membrane, which comparable to the positive control substance Eylea, even at a molar concentration only half of Eylea.

### Example 11. In vivo assay of the bispecific protein of the present invention in a rat nCNV model

Bispecific proteins were assayed in a rat nCNV model for three indicators, i.e., grade 4 fluorescence spot percentage, grade 4 fluorescence spot area and retinal thickness, on day 7, day 14 and day 21 after the injection. The results suggest that, for the choroidal neovascularization rat model under the experimental conditions of this example, the high-dose BiFpC2 group (15 µg/eye, single intravitreal injection) leads to a certain inhibition of neovascularization and leakage.

The present invention is not limited to the specific embodiments mentioned above. Any technical modifications made based on the technical solution of the present invention fall within the scope of protection of the present invention.

## Claims

1. A bispecific fusion polypeptide for binding a vascular endothelial growth factor (VEGF) A and VEGFC, comprising a first domain specifically recognizing VEGFA and a second domain specifically recognizing VEGFC.

2. The bispecific fusion polypeptide according to claim 1, **characterized in that**,
the first domain is a vascular endothelial growth factor receptor (VEGFR) specifically recognizing VEGFA or a functional fragment thereof, or is an antibody targeting VEGFA or a functional fragment thereof,
the second domain is a VEGFR specifically recognizing VEGFC or a functional fragment thereof, or is an antibody targeting VEGFC or a functional fragment thereof.

3. The bispecific fusion polypeptide according to claim 1 or 2, **characterized in that**, the first domain comprises immunoglobulin (Ig)-like domain 2 of VEGFR1 and Ig-like domain 3 of VEGFR2, or
the first domain comprises an antibody Fab fragment, a Fab' fragment, a F(ab')₂ fragment, an Fv fragment, a scFv fragment, a nanoantibody, a heavy chain variable region(VH) fragment or a light chain variable region (VL) fragment specifically binding to VEGFA.

4. The bispecific fusion polypeptide according to any one of claims 1-3, **characterized in that**, the second domain comprises Ig-like domain 1, Ig-like domain 2, and Ig-like domain 3 of VEGFR3, or
the second domain comprises an antibody Fab fragment, a Fab' fragment, a F(ab')₂ fragment, an Fv fragment, a scFv fragment, a nanoantibody, a VH fragment or a VL fragment specifically binding to VEGFC.

5. The bispecific fusion polypeptide according to any one of claims 1-4, **characterized in that**, the bispecific fusion polypeptide further comprises a third domain, wherein the third domain comprises an Fc region of a immunoglobulin,
preferably, the immunoglobulin is selected from IgA, IgG, IgM, IgD and IgE, preferably IgG, more preferably IgG1, IgG2, IgG3 or IgG4;
more preferably, the Fc region of a immunoglobulin has an amino acid sequence as shown in any one of SEQ ID NOs: 12-15, or an amino acid sequence having at least about 85%, 90%, 95%, 98%, 99% or 100% sequence identity to any one of SEQ ID NOs: 12-15.

6. The bispecific fusion polypeptide according to claim 5, **characterized in that**, the first domain, the second domain and/or the third domain connect to each other directly or via a linker,
preferably, the linker comprises at least 6 amino acids,
more preferably, the linker has an amino acid sequence as shown in SEQ ID NO: 7 or 8, or an amino acid sequence with one, two or three amino acids insertion, substitution or deletion compared to the amino acid sequence shown in SEQ ID NO: 7 or 8.

7. The bispecific fusion polypeptide according to any one of claims 1-6, **characterized in that**, the first domain comprises an amino acid sequence as shown in SEQ ID NO: 1 or an amino acid sequence having at least about 85%, 90%, 95%, 98%, 99% or 100% sequence identity to the amino acid sequence as shown in SEQ ID NO: 1, and an amino acid sequence as shown in SEQ ID NO: 2 or an amino acid sequence having at least about 85%, 90%, 95%, 98%, 99% or 100% sequence identity to the amino acid sequence as shown in SEQ ID NO: 2;
preferably, the first domain comprises an amino acid sequence as shown in SEQ ID NO: 9 or an amino acid sequence having at least about 85%, 90%, 95%, 98%, 99% or 100% sequence identity to the amino acid sequence as shown in SEQ ID NO: 9, or alternatively, the first domain comprises an amino acid sequence as shown in SEQ ID NO: 16 or an amino acid sequence having at least about 85%, 90%, 95%, 98%, 99% or 100% sequence identity to the amino acid sequence as shown in SEQ ID NO: 16.

8. The bispecific fusion polypeptide according to any one of claims 1-7, **characterized in that**, the second domain comprises:
an amino acid sequence as shown in SEQ ID NO: 3, an amino acid sequence corresponding to SEQ ID NO: 3 but with an amino acid at position 75 not being asparagine, or an amino acid sequence having at least about 85%, 90%, 95%, 98%, 99% or 100% sequence identity to the amino acid sequence as shown in SEQ ID NO: 3;
an amino acid sequence as shown in SEQ ID NO: 4 or an amino acid sequence having at least about 85%, 90%, 95%, 98%, 99% or 100% sequence identity to the amino acid sequence as shown in SEQ ID NO: 4 ; and
an amino acid sequence as shown in SEQ ID NO: 5 or an amino acid sequence having at least about 85%, 90%, 95%, 98%, 99% or 100% sequence identity to the amino acid sequence as shown in SEQ ID NO: 5.

9. The bispecific fusion polypeptide according to claim 8, **characterized in that**, in the amino acid sequence corresponding to SEQ ID NO: 3 but with an amino acid at position 75 not being asparagine, the amino acid at position 75 is replaced with glutamine, aspartic acid, glutamic acid, arginine or lysine;
preferably, the amino acid sequence corresponding to SEQ ID NO: 3 but with an amino acid at position 75 not being asparagine is an amino acid sequence as shown in SEQ ID NO: 6.

10. The bispecific fusion polypeptide according to any one of claims 1-9, **characterized in that**, the second domain comprises: an amino acid sequence as shown in SEQ ID NO: 10, an amino acid sequence corresponding to SEQ ID NO: 10 but with an amino acid at position 80 not being asparagine, or an amino acid sequence having at least about 85%, 90%, 95%, 98%, 99% or 100% sequence identity to the amino acid sequence as shown in SEQ ID NO: 10,
preferably, in the amino acid sequence corresponding to SEQ ID NO: 10 but with an amino acid at position 80 not being asparagine, the amino acid at position 80 is replaced with glutamine, aspartic acid, glutamic acid, arginine or lysine, more preferably, the amino acid sequence corresponding to SEQ ID NO: 10 but with an amino acid at position 80 not being asparagine is an amino acid sequence as shown in SEQ ID NO: 11.

11. The bispecific fusion polypeptide according to any one of claims 1-10, **characterized in that**, the bispecific fusion polypeptide comprises an amino acid sequence as shown in any one of SEQ ID NOs: 17-24, or an amino acid sequence having at least about 85%, 90%, 95%, 98%, 99% or 100% sequence identity to the amino acid sequence as shown in any one of SEQ ID NOs: 17-24.

12. An isolated nucleic acid molecule, which encodes the bispecific fusion polypeptide according to any one of claims 1-11.

13. A nucleic acid delivery vector, comprising the isolated nucleic acid molecule according to claim 12, preferably, the nucleic acid delivery vector comprises a nucleic acid delivery vector derived from adenovirus, adeno-associated virus, lentivirus or other acceptable nucleic acid delivery vectors.

14. A bispecific binding molecule for vascular endothelial growth factor (VEGF) A and VEGFC, **characterized in that**, the bispecific binding molecule comprises a dimer of the bispecific fusion polypeptide according to any one of claims 1-11, preferably, the dimer is a homodimer or a heterodimer.

15. A host cell, comprising the isolated nucleic acid molecule according to claim 12.

16. A pharmaceutical composition, comprising the bispecific fusion polypeptide according to any one of claims 1-11, the isolated nucleic acid molecule according to claim 12, the nucleic acid delivery vector according to claim 13, or the bispecific binding molecule according to claim 14, as well as a pharmaceutically acceptable carrier.

17. The pharmaceutical composition according to claim 16, **characterized in that**, the pharmaceutical composition is in the form of tablet, fine powder, granule, pill, injection, suspension, powder, emulsion, aerosol, gel, eye drop, sustained release formulation or sustained release implant.

18. A kit, comprising the pharmaceutical composition according to claim 16 or 17 enclosed in a container, wherein the container is preferably a glass ampoule, a glass vial, a plastic ampoule, a plastic vial, a plastic pouch or a pre-loaded syringe.

19. A method for treating or preventing diseases associated to VEGFA or VEGFC, comprising administering to a subject a therapeutically effective amount of the bispecific fusion polypeptide according to any one of claims 1-11, the isolated nucleic acid molecule according to claim 12, the nucleic acid delivery vector according to claim 13, the bispecific binding molecule according to claim 14, or the pharmaceutical composition according to claim 16 or 17.

20. The method according to claim 19, **characterized in that**, the disease is selected from angiogenic ophthalmopathy and cancer related indications, preferably, the disease is selected from age related macular degeneration, diabetic macular edema, diabetic retinopathy, angiogenic glaucoma, retinal vein occlusion, corneal neovascularization, neovascularization after corneal transplantation, breast cancer, kidney cancer, ovarian cancer, fallopian tube cancer, peritoneal cancer, gastric cancer, colorectal cancer, non-small cell lung cancer, bladder cancer, pancreatic cancer, liver cancer, cervical cancer and glioblastoma.
